(19) Europäisches Patentamt
European Patent Office
Office européen des brevets

(11) EP 1 514 922 A1

(12) EUROPEAN PATENT APPLICATION

(43) Date of publication:
**16.03.2005 Bulletin 2005/11**

(21) Application number: **03292246.0**

(22) Date of filing: **11.09.2003**

(51) Int Cl.[7]: **C12N 5/10**, C12N 15/82, A01H 5/00, A01K 67/027, C07K 14/405, C07K 19/00, C07K 7/04, C12N 15/62, G01N 33/84, G01N 33/542

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(71) Applicants:
- **INSTITUT PASTEUR 75015 Paris (FR)**
- **INSTITUT NATIONAL DE LA SANTE ET DE LA RECHERCHE MEDICALE (INSERM) 75013 Paris (FR)**

(72) Inventors:
- **Jacob, Yves 28130 Maintenon (FR)**
- **Brest, Pierre 94230 Cachan (FR)**
- **Real, Eléonore 92240 Malakoff (FR)**

(74) Representative:
**Callon de Lamarck, Jean-Robert et al**
**Cabinet Régimbeau**
**20, rue de Chazelles**
**75847 Paris cedex 17 (FR)**

# (54) Development of an anionic sensor for living cells: application to the human glycine receptor

(57) The subject of the invention is a polypeptide comprising a new mutant enhanced yellow fluorescent protein (EYFP) having anion binding properties, which may be further fused with an enhanced cyan fluorescent protein (ECFP) and further to a peptide linker inserted between said mutant EYFP and ECFP, thereby forming an anionic sensor, said anionic sensor may further be inserted in a transmembrane anionic channel. A complex comprising a transmembrane ionic channel and a polypeptidic biosensor is also disclosed. Also disclosed are nucleic acids and vectors, host cells and transgenic organisms thereof. Also disclosed herein are a method for preparing transformed cells capable of expressing ionic sensors at the vicinity of said ionic channels, methods for determining and monitoring dynamic changes in ion concentrations in cells, methods of selecting compounds capable of modifying said dynamic changes, sensing fluorescent microspheres and kits thereof.

EP 1 514 922 A1

SEQUENCE LISTING

<110> INSTITUT PASTEUR
INSTITUT NATIONAL DE LA SANTE ET DE LA RECHERCHE MEDICALE (INSERM)

<120> DEVELOPMENT OF AN ANIONIC SENSOR FOR LIVING CELLS: APPLICATION TO THE HUMAN GLYCINE

<130> D21407

<160> 13

<170> PatentIn version 3.2

<210> 1
<211> 240
<212> PRT
<213> artificial

<220>
<223> Protein sequence derived from the EYFP protein

<400> 1

```
Val Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Phe
    50                  55                  60

Gly Tyr Gly Leu Gln Cys Phe Ala Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Tyr Asn Ser His Asn Val Tyr Ile Met Ala Asp Lys Gln Lys Asn Gly
145                 150                 155                 160

Ile Lys Val Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190
```

```
Val Leu Leu Pro Asp Asn His Tyr Leu Ser Tyr Gln Ser Ala Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys Gly Ser
225                 230                 235                 240


<210> 2
<211> 241
<212> PRT
<213> artificial

<220>
<223> Protein sequence derived from the ECFP protein

<400> 2

Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Trp Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Ile Ser His Asn Val Tyr Ile Thr Ala Asp Lys Gln Lys Asn
145                 150                 155                 160

Gly Ile Lys Ala Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
```

```
    210                 215                 220

Val Thr Ala Ala Gly Ile Thr Leu Gly Met Asp Glu Leu Tyr Lys Ser
225                 230                 235                 240

Gly


<210> 3
<211> 24
<212> PRT
<213> artificial

<220>
<223> Peptide linker 1

<400> 3

Lys Leu Thr Gly Ser Gly Ser Gly Glu Asn Leu Tyr Phe Gln Gly Gly
1               5                   10                  15

Gly Ser Gly Gly Thr Ser Ser Thr
            20


<210> 4
<211> 20
<212> PRT
<213> artificial

<220>
<223> Peptide linker 2

<400> 4

Gly Ser Gly Ser Gly Glu Asn Leu Tyr Phe Gln Gly Gly Gly Ser Gly
1               5                   10                  15

Gly Ser Gly Ser
            20


<210> 5
<211> 501
<212> PRT
<213> artificial

<220>
<223> Anionic sensor protein sequence

<400> 5

Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45
```

Cys Thr Thr Gly Lys Leu Pro Val Pro Trp Pro Thr Leu Val Thr Thr
    50                  55                  60

Leu Thr Trp Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Ile Ser His Asn Val Tyr Ile Thr Ala Asp Lys Gln Lys Asn
145                 150                 155                 160

Gly Ile Lys Ala Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys Ser
225                 230                 235                 240

Gly Gly Ser Gly Ser Gly Glu Asn Leu Tyr Phe Gln Gly Gly Gly Ser
                245                 250                 255

Gly Gly Ser Gly Ser Val Ser Lys Gly Glu Glu Leu Phe Thr Gly Val
            260                 265                 270

Val Pro Ile Leu Val Glu Leu Asp Gly Asp Val Asn Gly His Lys Phe
        275                 280                 285

Ser Val Ser Gly Glu Gly Glu Gly Asp Ala Thr Tyr Gly Lys Leu Thr
    290                 295                 300

Leu Lys Phe Ile Cys Thr Thr Gly Lys Leu Pro Val Pro Trp Pro Thr
305                 310                 315                 320

Leu Val Thr Thr Phe Gly Tyr Gly Leu Gln Cys Phe Ala Arg Tyr Pro
                325                 330                 335

Asp His Met Lys Gln His Asp Phe Phe Lys Ser Ala Met Pro Glu Gly
            340                 345                 350

Tyr Val Gln Glu Arg Thr Ile Phe Phe Lys Asp Asp Gly Asn Tyr Lys
        355                 360                 365

Thr Arg Ala Glu Val Lys Phe Glu Gly Asp Thr Leu Val Asn Arg Ile
    370                 375                 380

Glu Leu Lys Gly Ile Asp Phe Lys Glu Asp Gly Asn Ile Leu Gly His
385                 390                 395                 400

Lys Leu Glu Tyr Asn Tyr Asn Ser Gln Asn Val Tyr Leu Met Ala Asp
                405                 410                 415

Lys Gln Lys Asn Gly Ile Lys Ser Asn Phe Lys Ile Arg His Asn Ile
            420                 425                 430

Glu Asp Gly Ser Val Gln Leu Ala Asp His Tyr Gln Gln Asn Thr Pro
        435                 440                 445

Ile Gly Asp Gly Pro Val Leu Leu Pro Asp Asn His Tyr Leu Ser Tyr
    450                 455                 460

Gln Ser Ala Leu Ser Lys Asp Pro Asn Glu Lys Arg Asp His Met Val
465                 470                 475                 480

Leu Leu Glu Phe Val Thr Ala Ala Gly Ile Thr Leu Gly Met Asp Glu
                485                 490                 495

Leu Tyr Lys Gly Ser
            500

<210> 6
<211> 449
<212> PRT
<213> artificial

<220>
<223> Protein sequence derived from the human glycine receptor-alpha1 sub-unit protein

<400> 6

Met Tyr Ser Phe Asn Thr Leu Arg Leu Tyr Leu Ser Gly Ala Ile Val
1               5                   10                  15

Phe Phe Ser Leu Ala Ala Ser Lys Glu Ala Glu Ala Ala Arg Ser Ala
            20                  25                  30

Thr Lys Pro Met Ser Pro Ser Asp Phe Leu Asp Lys Leu Met Gly Arg
        35                  40                  45

Thr Ser Gly Tyr Asp Ala Arg Ile Arg Pro Asn Phe Lys Gly Pro Pro
    50                  55                  60

Val Asn Val Ser Cys Asn Ile Phe Ile Asn Ser Phe Gly Ser Ile Ala
65                  70                  75                  80

Glu Thr Thr Met Asp Tyr Arg Val Asn Ile Phe Leu Arg Gln Gln Trp
                85                  90                  95

Asn Asp Pro Arg Leu Ala Tyr Asn Glu Tyr Pro Asp Asp Ser Leu Asp
            100                 105                 110

Leu Asp Pro Ser Met Leu Asp Ser Ile Trp Lys Pro Asp Leu Phe Phe

115 120 125

Ala Asn Glu Lys Gly Ala His Phe His Glu Ile Thr Thr Asp Asn Lys
130 135 140

Leu Leu Arg Ile Ser Arg Asn Gly Asn Val Leu Tyr Ser Ile Arg Ile
145 150 155 160

Thr Leu Thr Leu Ala Cys Pro Met Asp Leu Lys Asn Phe Pro Met Asp
165 170 175

Val Gln Thr Cys Ile Met Gln Leu Glu Ser Phe Gly Tyr Thr Met Asn
180 185 190

Asp Leu Ile Phe Glu Trp Gln Glu Gln Gly Ala Val Gln Val Ala Asp
195 200 205

Gly Leu Thr Leu Pro Gln Phe Ile Leu Lys Glu Glu Lys Asp Leu Arg
210 215 220

Tyr Cys Thr Lys His Tyr Asn Thr Gly Lys Phe Thr Cys Ile Glu Ala
225 230 235 240

Arg Phe His Leu Glu Arg Gln Met Gly Tyr Tyr Leu Ile Gln Met Tyr
245 250 255

Ile Pro Ser Leu Leu Ile Val Ile Leu Ser Trp Ile Ser Phe Trp Ile
260 265 270

Asn Met Asp Ala Ala Pro Ala Arg Val Gly Leu Gly Ile Thr Thr Val
275 280 285

Leu Thr Met Thr Thr Gln Ser Ser Gly Ser Arg Ala Ser Leu Pro Lys
290 295 300

Val Ser Tyr Val Lys Ala Ile Asp Ile Trp Met Ala Val Cys Leu Leu
305 310 315 320

Phe Val Phe Ser Ala Leu Leu Glu Tyr Ala Ala Val Asn Phe Val Ser
325 330 335

Arg Gln His Lys Glu Leu Leu Arg Phe Arg Arg Lys Arg Arg His His
340 345 350

Lys Glu Asp Glu Ala Gly Glu Gly Arg Phe Asn Phe Ser Ala Tyr Gly
355 360 365

Met Gly Pro Ala Cys Leu Gln Ala Lys Asp Gly Ile Ser Val Lys Gly
370 375 380

Ala Asn Asn Ser Asn Thr Thr Asn Pro Pro Pro Ala Pro Ser Lys Ser
385 390 395 400

Pro Glu Glu Met Arg Lys Leu Phe Ile Gln Arg Ala Lys Lys Ile Asp
405 410 415

Lys Ile Ser Arg Ile Gly Phe Pro Met Ala Phe Leu Ile Phe Asn Met
420 425 430

Phe Tyr Trp Ile Ile Tyr Lys Ile Val Arg Arg Glu Asp Val His Asn
435 440 445

Gln

<210> 7
<211> 957
<212> PRT
<213> artificial

<220>
<223> Protein sequence of the glycine receptor-anionic sensor

<400> 7

```
Met Tyr Ser Phe Asn Thr Leu Arg Leu Tyr Leu Ser Gly Ala Ile Val
1               5                   10                  15

Phe Phe Ser Leu Ala Ala Ser Lys Glu Ala Glu Ala Ala Arg Ser Ala
            20                  25                  30

Thr Lys Pro Met Ser Pro Ser Asp Phe Leu Asp Lys Leu Met Gly Arg
        35                  40                  45

Thr Ser Gly Tyr Asp Ala Arg Ile Arg Pro Asn Phe Lys Gly Pro Pro
    50                  55                  60

Val Asn Val Ser Cys Asn Ile Phe Ile Asn Ser Phe Gly Ser Ile Ala
65                  70                  75                  80

Glu Thr Thr Met Asp Tyr Arg Val Asn Ile Phe Leu Arg Gln Gln Trp
                85                  90                  95

Asn Asp Pro Arg Leu Ala Tyr Asn Glu Tyr Pro Asp Asp Ser Leu Asp
            100                 105                 110

Leu Asp Pro Ser Met Leu Asp Ser Ile Trp Lys Pro Asp Leu Phe Phe
        115                 120                 125

Ala Asn Glu Lys Gly Ala His Phe His Glu Ile Thr Thr Asp Asn Lys
    130                 135                 140

Leu Leu Arg Ile Ser Arg Asn Gly Asn Val Leu Tyr Ser Ile Arg Ile
145                 150                 155                 160

Thr Leu Thr Leu Ala Cys Pro Met Asp Leu Lys Asn Phe Pro Met Asp
                165                 170                 175

Val Gln Thr Cys Ile Met Gln Leu Glu Ser Phe Gly Tyr Thr Met Asn
            180                 185                 190

Asp Leu Ile Phe Glu Trp Gln Glu Gln Gly Ala Val Gln Val Ala Asp
        195                 200                 205

Gly Leu Thr Leu Pro Gln Phe Ile Leu Lys Glu Glu Lys Asp Leu Arg
    210                 215                 220

Tyr Cys Thr Lys His Tyr Asn Thr Gly Lys Phe Thr Cys Ile Glu Ala
225                 230                 235                 240

Arg Phe His Leu Glu Arg Gln Met Gly Tyr Tyr Leu Ile Gln Met Tyr
```

```
            245                 250                 255
Ile Pro Ser Leu Leu Ile Val Ile Leu Ser Trp Ile Ser Phe Trp Ile
        260                 265                 270

Asn Met Asp Ala Ala Pro Ala Arg Val Gly Leu Gly Ile Thr Thr Val
    275                 280                 285

Leu Thr Met Thr Thr Gln Ser Ser Gly Ser Arg Ala Ser Leu Pro Lys
    290                 295                 300

Val Ser Tyr Val Lys Ala Ile Asp Ile Trp Met Ala Val Cys Leu Leu
305                 310                 315                 320

Phe Val Phe Ser Ala Leu Leu Glu Tyr Ala Ala Val Asn Phe Val Ser
                325                 330                 335

Arg Gln His Lys Glu Leu Leu Arg Phe Arg Arg Lys Arg Arg His His
            340                 345                 350

Lys Glu Asp Glu Ala Gly Glu Gly Arg Phe Asn Phe Ser Ala Tyr Gly
        355                 360                 365

Met Gly Pro Ala Cys Leu Gln Ala Lys Asp Gly Ile Ser Val Lys Gly
    370                 375                 380

Ala Asn Asn Ser Asn Thr Thr Asn Pro Pro Leu Ala Leu Pro Val Ala
385                 390                 395                 400

Thr Met Val Ser Lys Gly Glu Glu Leu Phe Thr Gly Val Val Pro Ile
                405                 410                 415

Leu Val Glu Leu Asp Gly Asp Val Asn Gly His Lys Phe Ser Val Ser
            420                 425                 430

Gly Glu Gly Glu Gly Asp Ala Thr Tyr Gly Lys Leu Thr Leu Lys Phe
        435                 440                 445

Ile Cys Thr Thr Gly Lys Leu Pro Val Pro Trp Pro Thr Leu Val Thr
    450                 455                 460

Thr Leu Thr Trp Gly Val Gln Cys Phe Ser Arg Tyr Pro Asp His Met
465                 470                 475                 480

Lys Gln His Asp Phe Phe Lys Ser Ala Met Pro Glu Gly Tyr Val Gln
                485                 490                 495

Glu Arg Thr Ile Phe Phe Lys Asp Asp Gly Asn Tyr Lys Thr Arg Ala
            500                 505                 510

Glu Val Lys Phe Glu Gly Asp Thr Leu Val Asn Arg Ile Glu Leu Lys
        515                 520                 525

Gly Ile Asp Phe Lys Glu Asp Gly Asn Ile Leu Gly His Lys Leu Glu
    530                 535                 540

Tyr Asn Tyr Ile Ser His Asn Val Tyr Ile Thr Ala Asp Lys Gln Lys
545                 550                 555                 560

Asn Gly Ile Lys Ala Asn Phe Lys Ile Arg His Asn Ile Glu Asp Gly
                565                 570                 575
```

Ser Val Gln Leu Ala Asp His Tyr Gln Gln Asn Thr Pro Ile Gly Asp
580 585 590

Gly Pro Val Leu Leu Pro Asp Asn His Tyr Leu Ser Thr Gln Ser Ala
595 600 605

Leu Ser Lys Asp Pro Asn Glu Lys Arg Asp His Met Val Leu Leu Glu
610 615 620

Phe Val Thr Ala Ala Gly Ile Thr Leu Gly Met Asp Glu Leu Tyr Lys
625 630 635 640

Ser Gly Gly Ser Gly Ser Gly Glu Asn Leu Tyr Phe Gln Gly Gly Gly
645 650 655

Ser Gly Gly Ser Gly Ser Val Ser Lys Gly Glu Glu Leu Phe Thr Gly
660 665 670

Val Val Pro Ile Leu Val Glu Leu Asp Gly Asp Val Asn Gly His Lys
675 680 685

Phe Ser Val Ser Gly Glu Gly Glu Gly Asp Ala Thr Tyr Gly Lys Leu
690 695 700

Thr Leu Lys Phe Ile Cys Thr Thr Gly Lys Leu Pro Val Pro Trp Pro
705 710 715 720

Thr Leu Val Thr Thr Phe Gly Tyr Gly Leu Gln Cys Phe Ala Arg Tyr
725 730 735

Pro Asp His Met Lys Gln His Asp Phe Phe Lys Ser Ala Met Pro Glu
740 745 750

Gly Tyr Val Gln Glu Arg Thr Ile Phe Phe Lys Asp Asp Gly Asn Tyr
755 760 765

Lys Thr Arg Ala Glu Val Lys Phe Glu Gly Asp Thr Leu Val Asn Arg
770 775 780

Ile Glu Leu Lys Gly Ile Asp Phe Lys Glu Asp Gly Asn Ile Leu Gly
785 790 795 800

His Lys Leu Glu Tyr Asn Tyr Asn Ser Gln Asn Val Tyr Leu Met Ala
805 810 815

Asp Lys Gln Lys Asn Gly Ile Lys Ser Asn Phe Lys Ile Arg His Asn
820 825 830

Ile Glu Asp Gly Ser Val Gln Leu Ala Asp His Tyr Gln Gln Asn Thr
835 840 845

Pro Ile Gly Asp Gly Pro Val Leu Leu Pro Asp Asn His Tyr Leu Ser
850 855 860

Tyr Gln Ser Ala Leu Ser Lys Asp Pro Asn Glu Lys Arg Asp His Met
865 870 875 880

Val Leu Leu Glu Phe Val Thr Ala Ala Gly Ile Thr Leu Gly Met Asp
885 890 895

Glu Leu Tyr Lys Gly Ser Pro Ala Pro Ser Lys Ser Pro Glu Glu Met
900 905 910

Arg Lys Leu Phe Ile Gln Arg Ala Lys Lys Ile Asp Lys Ile Ser Arg
915 920 925

Ile Gly Phe Pro Met Ala Phe Leu Ile Phe Asn Met Phe Tyr Trp Ile
930 935 940

Ile Tyr Lys Ile Val Arg Arg Glu Asp Val His Asn Gln
945 950 955

<210> 8
<211> 720
<212> DNA
<213> artificial

<220>
<223> Nucleotidic sequence encoding the protein sequence derived from the EYFP protein

<400> 8
gtgagcaagg gcgaggagct gttcaccggg gtggtgccca tcctggtcga gctggacggc 60
gacgtaaacg gccacaagtt cagcgtgtcc ggcgagggcg agggcgatgc cacctacggc 120
aagctgaccc tgaagttcat ctgcaccacc ggcaagctgc ccgtgccctg gcccaccctc 180
gtgaccacct tcggctacgg cctgcagtgc ttcgcccgct accccgacca catgaagcag 240
cacgacttct tcaagtccgc catgcccgaa ggctacgtcc aggagcgcac catcttcttc 300
aaggacgacg gcaactacaa gacccgcgcc gaggtgaagt tcgagggcga caccctggtg 360
aaccgcatcg agctgaaggg catcgacttc aaggaggacg gcaacatcct ggggcacaag 420
ctggagtaca actacaacag ccacaacgtc tatatcatgg ccgacaagca gaagaacggc 480
atcaaggtga acttcaagat ccgccacaac atcgaggacg gcagcgtgca gctcgccgac 540
cactaccagc agaacacccc catcggcgac ggccccgtgc tgctgcccga caaccactac 600
ctgagctacc agtccgccct gagcaaagac cccaacgaga agcgcgatca catggtcctg 660
ctggagttcg tgaccgccgc cgggatcact ctcggcatgg acgagctgta caagggatct 720

<210> 9
<211> 723
<212> DNA
<213> artificial

<220>
<223> Nucleotidic sequence encoding the protein sequence derived from the ECFP protein

<400> 9
atggtgagca agggcgagga gctgttcacc ggggtggtgc ccatcctggt cgagctggac 60
ggcgacgtaa acggccacaa gttcagcgtg tccggcgagg gcgagggcga tgccacctac 120
ggcaagctga ccctgaagtt catctgcacc accggcaagc tgcccgtgcc ctggcccacc 180
ctcgtgacca ccctgacctg gggcgtgcag tgcttcagcc gctaccccga ccacatgaag 240
cagcacgact tcttcaagtc cgccatgccc gaaggctacg tccaggagcg caccatcttc 300
ttcaaggacg acggcaacta caagacccgc gccgaggtga agttcgaggg cgacaccctg 360
gtgaaccgca tcgagctgaa gggcatcgac ttcaaggagg acggcaacat cctggggcac 420
aagctggagt acaactacat cagccacaac gtctatatca ccgccgacaa gcagaagaac 480
ggcatcaagg ccaacttcaa gatccgccac aacatcgagg acggcagcgt gcagctcgcc 540
gaccactacc agcagaacac ccccatcggc gacggccccg tgctgctgcc cgacaaccac 600
tacctgagca cccagtccgc cctgagcaaa gaccccaacg agaagcgcga tcacatggtc 660
ctgctggagt tcgtgaccgc cgccgggatc actctcggca tggacgagct gtacaagtcc 720
gga 723

<210> 10
<211> 60
<212> DNA
<213> artificial

<220>
<223> Nucleotidic sequence encoding the peptide linker 2

<400> 10
ggtagtggca gtggtgagaa tttgtacttc caaggaggcg gcagcggagg cagcggatct 60

<210> 11
<211> 1503
<212> DNA
<213> artificial

<220>
<223> Nucleotidic sequence encoding the anionic sensor protein

<400> 11
atggtgagca agggcgagga gctgttcacc ggggtggtgc ccatcctggt cgagctggac 60
ggcgacgtaa acggccacaa gttcagcgtg tccggcgagg gcgagggcga tgccacctac 120
ggcaagctga ccctgaagtt catctgcacc accggcaagc tgcccgtgcc ctggcccacc 180
ctcgtgacca ccctgacctg gggcgtgcag tgcttcagcc gctaccccga ccacatgaag 240
cagcacgact tcttcaagtc cgccatgccc gaaggctacg tccaggagcg caccatcttc 300
ttcaaggacg acggcaacta caagacccgc gccgaggtga agttcgaggg cgacaccctg 360
gtgaaccgca tcgagctgaa gggcatcgac ttcaaggagg acggcaacat cctggggcac 420
aagctggagt acaactacat cagccacaac gtctatatca ccgccgacaa gcagaagaac 480
ggcatcaagg ccaacttcaa gatccgccac aacatcgagg acggcagcgt gcagctcgcc 540
gaccactacc agcagaacac ccccatcggc gacggccccg tgctgctgcc cgacaaccac 600
tacctgagca cccagtccgc cctgagcaaa gaccccaacg agaagcgcga tcacatggtc 660
ctgctggagt tcgtgaccgc cgccgggatc actctcggca tggacgagct gtacaagtcc 720
ggaggtagtg gcagtggtga gaatttgtac ttccaaggag gcggcagcgg aggcagcgga 780
tctgtgagca agggcgagga gctgttcacc ggggtggtgc ccatcctggt cgagctggac 840
ggcgacgtaa acggccacaa gttcagcgtg tccggcgagg gcgagggcga tgccacctac 900
ggcaagctga ccctgaagtt catctgcacc accggcaagc tgcccgtgcc ctggcccacc 960
ctcgtgacca ccttcggcta cggcctgcag tgcttcgccc gctaccccga ccacatgaag 1020
cagcacgact tcttcaagtc cgccatgccc gaaggctacg tccaggagcg caccatcttc 1080
ttcaaggacg acggcaacta caagacccgc gccgaggtga agttcgaggg cgacaccctg 1140
gtgaaccgca tcgagctgaa gggcatcgac ttcaaggagg acggcaacat cctggggcac 1200
aagctggagt acaactacaa cagccagaac gtctatctca tggccgacaa gcagaagaac 1260
ggcatcaagt cgaacttcaa gatccgccac aacatcgagg acggcagcgt gcagctcgcc 1320
gaccactacc agcagaacac ccccatcggc gacggccccg tgctgctgcc cgacaaccac 1380
tacctgagct accagtccgc cctgagcaaa gaccccaacg agaagcgcga tcacatggtc 1440
ctgctggagt tcgtgaccgc cgccgggatc actctcggca tggacgagct gtacaaggga 1500
tct 1503

<210> 12
<211> 1419
<212> DNA
<213> artificial

<220>
<223> Nucleotidic sequence encoding the protein sequence derived
from the human glycin receptor-alpha1 sub-unit protein

<400> 12
atgtacagct tcaatactct tcgactctac ctttcgggag ccattgtatt cttcagcctt 60
gctgcttcta aggaggctga agctgctcgc tccgcaacca agcctatgtc accctcggat 120
ttcctggata agctaatggg gagaacctcc ggatatgatg ccaggatcag gcccaatttt 180

aaaggtcccc cagtgaacgt gagctgcaac attttcatca acagctttgg ttccattgct 240
gagacaacca tggactatag ggtcaacatc ttcctgcggc agcaatggaa cgacccccgc 300
ctggcctata atgaataccc tgacgactct ctggacctgg acccatccat gctggactcc 360
atctggaaac ctgacctgtt ctttgccaac gagaaggggg cccacttcca tgagatcacc 420
acagacaaca aattgctaag gatctcccgg aatgggaatg tcctctacag catcagaatc 480
accctgacac tggcctgccc catggacttg aagaatttcc ccatggatgt ccagacatgt 540
atcatgcaac tggaaagctt tggatatacg atgaatgacc tcatctttga gtggcaggaa 600
cagggagccg tgcaggtagc agatggacta actctgcccc agtttatctt gaaggaagag 660
aaggacttga gatactgcac caagcactac aacacaggta aattcacctg cattgaggcc 720
cggttccacc tggagcggca gatgggttac tacctgattc agatgtatat tcccagcctg 780
ctcattgtca tcctctcatg gatctccttc tggatcaaca tggatgctgc acctgctcgt 840
gtgggcctag gcatcaccac tgtgctcacc atgaccaccc agagctccgg ctctcgagca 900
tctctgccca aggtgtccta tgtgaaagcc attgacattt ggatggcagt ttgcctgctc 960
tttgtgttct cagccctatt agaatatgct gccgttaact ttgtgtctcg gcaacataag 1020
gagctgctcc gattcaggag gaagcggaga catcacaagg aggatgaagc tggagaaggc 1080
cgctttaact tctctgccta tgggatgggc ccagcctgtc tacaggccaa ggatggcatc 1140
tcagtcaagg gcgccaacaa cagtaacacc accaaccccc ctcctgcacc atctaagtcc 1200
ccagaggaga tgcgaaaact cttcatccag agggccaaga agatcgacaa aatatcccgc 1260
attggcttcc ccatggcctt cctcattttc aacatgttct actggatcat ctacaagatt 1320
gtccgtagag aggacgtcca caaccagtga agggtctgaa aggttggggg aggctgggag 1380
aggggaacgt gggaatagca caggaatctg agagacggt 1419

<210> 13
<211> 2877
<212> DNA
<213> artificial

<220>
<223> Nucleotidic sequence encoding the protein sequence of the glycine receptor-anionic sensor

<400> 13
atgtacagct tcaatactct tcgactctac ctttcgggag ccattgtatt cttcagcctt 60
gctgcttcta aggaggctga agctgctcgc tccgcaacca agcctatgtc accctcggat 120
ttcctggata agctaatggg gagaacctcc ggatatgatg ccaggatcag gcccaatttt 180
aaaggtcccc cagtgaacgt gagctgcaac attttcatca acagctttgg ttccattgct 240
gagacaacca tggactatag ggtcaacatc ttcctgcggc agcaatggaa cgacccccgc 300
ctggcctata atgaataccc tgacgactct ctggacctgg acccatccat gctggactcc 360
atctggaaac ctgacctgtt ctttgccaac gagaaggggg cccacttcca tgagatcacc 420
acagacaaca aattgctaag gatctcccgg aatgggaatg tcctctacag catcagaatc 480
accctgacac tggcctgccc catggacttg aagaatttcc ccatggatgt ccagacatgt 540
atcatgcaac tggaaagctt tggatatacg atgaatgacc tcatctttga gtggcaggaa 600
cagggagccg tgcaggtagc agatggacta actctgcccc agtttatctt gaaggaagag 660
aaggacttga gatactgcac caagcactac aacacaggta aattcacctg cattgaggcc 720
cggttccacc tggagcggca gatgggttac tacctgattc agatgtatat tcccagcctg 780
ctcattgtca tcctctcatg gatctccttc tggatcaaca tggatgctgc acctgctcgt 840
gtgggcctag gcatcaccac tgtgctcacc atgaccaccc agagctccgg ctctcgagca 900
tctctgccca aggtgtccta tgtgaaagcc attgacattt ggatggcagt ttgcctgctc 960
tttgtgttct cagccctatt agaatatgct gccgttaact ttgtgtctcg gcaacataag 1020
gagctgctcc gattcaggag gaagcggaga catcacaagg aggatgaagc tggagaaggc 1080
cgctttaact tctctgccta tgggatgggc ccagcctgtc tacaggccaa ggatggcatc 1140
tcagtcaagg gcgccaacaa cagtaacacc accaaccccc ctctagcgct accggtcgcc 1200
accatggtga gcaagggcga ggagctgttc accggggtgg tgcccatcct ggtcgagctg 1260
gacggcgacg taaacggcca caagttcagc gtgtccggcg agggcgaggg cgatgccacc 1320
tacggcaagc tgaccctgaa gttcatctgc accaccggca agctgcccgt gccctggccc 1380
accctcgtga ccaccctgac ctggggcgtg cagtgcttca gccgctaccc cgaccacatg 1440
aagcagcacg acttcttcaa gtccgccatg cccgaaggct acgtccagga gcgcaccatc 1500
ttcttcaagg acgacggcaa ctacaagacc cgcgccgagg tgaagttcga gggcgacacc 1560
ctggtgaacc gcatcgagct gaagggcatc gacttcaagg aggacggcaa catcctgggg 1620
cacaagctgg agtacaacta catcagccac aacgtctata tcaccgccga caagcagaag 1680

```
aacggcatca aggccaactt caagatccgc cacaacatcg aggacggcag cgtgcagctc   1740
gccgaccact accagcagaa cacccccatc ggcgacggcc ccgtgctgct gcccgacaac   1800
cactacctga gcacccagtc cgccctgagc aaagacccca acgagaagcg cgatcacatg   1860
gtcctgctgg agttcgtgac cgccgccggg atcactctcg gcatggacga gctgtacaag   1920
tccggaggta gtggcagtgg tgagaatttg tacttccaag gaggcggcag cggaggcagc   1980
ggatctgtga gcaagggcga ggagctgttc accggggtgg tgcccatcct ggtcgagctg   2040
gacggcgacg taaacggcca caagttcagc gtgtccggcg agggcgaggg cgatgccacc   2100
tacggcaagc tgaccctgaa gttcatctgc accaccggca agctgcccgt gccctggccc   2160
accctcgtga ccaccttcgg ctacggcctg cagtgcttcg cccgctaccc cgaccacatg   2220
aagcagcacg acttcttcaa gtccgccatg cccgaaggct acgtccagga gcgcaccatc   2280
ttcttcaagg acgacggcaa ctacaagacc cgcgccgagg tgaagttcga gggcgacacc   2340
ctggtgaacc gcatcgagct gaagggcatc gacttcaagg aggacggcaa catcctgggg   2400
cacaagctgg agtacaacta caacagccag aacgtctatc tcatggccga caagcagaag   2460
aacggcatca agtcgaactt caagatccgc cacaacatcg aggacggcag cgtgcagctc   2520
gccgaccact accagcagaa cacccccatc ggcgacggcc ccgtgctgct gcccgacaac   2580
cactacctga gctaccagtc cgccctgagc aaagacccca acgagaagcg cgatcacatg   2640
gtcctgctgg agttcgtgac cgccgccggg atcactctcg gcatggacga gctgtacaag   2700
ggatctcctg caccatctaa gtccccagag gagatgcgaa aactcttcat ccagagggcc   2760
aagaagatcg acaaaatatc ccgcattggc ttccccatgg ccttcctcat tttcaacatg   2820
ttctactgga tcatctacaa gattgtccgt agagaggacg tccacaacca gtgataa      2877
```

## Description

The subject of the invention is a polypeptide comprisisng a new mutant enhanced yellow fluorescent protein (EYFP) having anion binding properties, which may be further fused with an enhanced cyan fluorescent protein (ECFP) and further to a peptide linker inserted between said mutant EYFP and ECFP, thereby forming an anionic sensor, said anionic sensor may further be inserted in a transmembrane anionic channel. A complex comprising a transmembrane ionic channel and a polypeptidic biosensor is also disclosed. Also disclosed are nucleic acids and vectors, host cells and transgenic organisms thereof. Also disclosed herein are a method for preparing transformed cells capable of expressing ionic sensors at the vicinity of said ionic channels, methods for determining and monitoring dynamic changes in ion concentrations in cells, methods of selecting compounds capable of modifying said dynamic changes, sensing fluorescent microspheres and kits thereof.

Chloride (Cl-) is by far the most abundant physiological anion. It is present in every cell of biological organisms and participates in a variety of physiological functions. The most important of them are control of membrane excitability, cell volume and intracellular pH regulation, and transepithelial transport (Hille B., Sinauer Associates Inc, 1992). This ion is also crucial for nervous system functioning, as the main inhibitory drive in central and peripheral neuronal circuits is provided by GABAergic and glycinergic synapses, which operate through chloride permeability regulation. Dynamic changes in intracellular $Cl^-$ concentration is also a key mechanism of signal transduction regulation in olfactory neurons (Nakamura et al., 1997, Neurosci Lett 237:5-8), astrocytes (Bekar and Walz, 1999) and in other cell types (Cherubini et al., 1991, *14*, 515-519; Wagner et al., 1997).

Intracellular chloride concentration ($[Cl^-]_i$) and its permeability is highly regulated by a variety of $Cl^-$-selective channels and $Cl^-$ transporters. Dysfunction of these proteins results in the occasion of various diseases. For instance, the most prevalent lethal genetic disease, cystic fibrosis (Kerem et al., 1989, Science *245*, 1073-1080) arises from mutation in the specific regulator of $Cl^-$ permeability: cystic fibrosis transmembrane conductance regulator protein (CFTR). This voltage-independent $Cl^-$ channel is found in the epithelial cells of many tissues (intestine, lung, reproductive tract, pancreatic ducts). Mutation in the gene encoding CFTR affects 1 in 2000-2500 people (Aschcroft, 2000, Academis Press). Several other human diseases have been linked to dysfunctional $Cl^-$ homeostasis: myotonia congenita (Koch et al., 1992, Science *257*, 797-800), congenital chloride diarrhoea (Kere et al., 1999, AmJ Physiol 276: G7-G13), inherited hypercalciuric nephrolithiasis (Lloyd et al., 1996, Nature *379*, 445-49), Bartter's and Gitelman's syndromes (Simon and Lifton, 1996, Am J Physiol 271:F961-F966), and hyperekplexia/startle disease (Shiang et al., 1993).

Despite the importance of $Cl^-$ for many cellular functions, there is little knowledge available on the mechanisms regulating the $[Cl^-]_i$ in healthy conditions and at diseases. This lack of information is primarily due to technical difficulties in the measurement of chloride fluxes in living cells. Several methods were proposed to monitor $[Cl^-]_i$. First, the method using $Cl^-$- selective microelectrodes (Thomas, 1978, London: Academic Press; Kaila, 1994, Prog Neurobiol 42:489-537; Krapf et al., 1988, Biophys J 53:955-962) is only appropriate for cells with large cell size. In addition, it is slow and recordings are often distorted by the interference of the electrode with intracellular anions and anion leakage from the reference barrel (Ballanyi, 1984, Neuroscience 12:917-927).

Second, there exists a method of monitoring of $[Cl^-]_i$ using quinoline-based fluorescent dyes sensitive to $Cl^-$ such as 6-methoxy-N-(3-sulphopropyl)quinolinium (SPQ), MQAE or 6-methoxy-N-ethylquinolinium chloride (MEQ). These dyes have been used for measurements of $[Cl^-]_i$ in a variety of preparations, including isolated growth cones (Fukura et al., 1996, J Neurochem 67:1426-1434), neurons (Dallwig et al., 1999, Pflugers Arch 437:289-297; Engblom et al., 1989, Neurosci Lett 104:326-330; Frech et al., 1999, J Neurobiol 40:386-396; Schwartz and Yu , 1995, J Neurosci Methods 62:185-192), glia (Bevensee et al., 1997, J Gen Physiol 110:467-483), different types of epithelial cells (Krapf et al., 1988; Lau et al., 1994, Pflugers Arch 427:24-32), fibroblasts (Chao et al., 1989, Biophys J 56:1071-1081 ; Woll et al., 1999) and pancreatic beta-cells (Eberhardson et al., 2000, Cell Signal 12:781-786). Quinolinium compounds have low biological toxicity, relatively good sensitivity and selectivity to $Cl^-$, and rapid response to changes in $[Cl^-]$. The major disadvantage of all quinoline-based indicator dyes is, that they are prone to unusually strong bleaching (Inglefield and Schwartz-Bloom, 1997, J Neurosci Methods 75:127-135; Nakamura et al., 1997). This restricts the duration of the measurements and allows only a very low data acquisition rate (0.2-2 frames per minute) (Fukuda et al., 1998, Neurosci Res 32:363-371; Inglefield and Schwartz-Bloom, 1997; Sah R, Schwartz-Bloom, 1999, J Neurosci 19:9209-9217).

Several years ago a mutant form of green fluorescent protein (GFP) was designed, which contains four point mutations and has red-shifted excitation and emission spectra compared with GFP (Ormo and al., Science. 1996 Sep 6;273(5280):1392-5. ; Elsliger and al., Biochemistry. 1999 Apr 27;38(17):5296-301). It was found that the fluorescence of this yellow fluorescent variant of the GFP, called yellow fluorescent protein (YFP), is sensitive to pH and also to various anions, including chloride (Wachter and Remington, Curr Biol. 1999 Sep 9;9(17):R628-9). This enabled a third method for anion concentration monitoring, which is based on the halide binding properties of YFP (Galietta and al., 2001 FEBS Letter 499, 220-224; Jayaraman and al., J Biol Chem. 2000 Mar 3;275(9):6047-50).

But a major drawback of this YFP in measurement of intracellular anion concentrations, such as $[Cl^-]_i$ , is that

its sensitivity to this anion is not appropriate with the animal and plant intracellular chloride concentration, which is less than 40 mM. Indeed, at physiological pH (7,5), for YFP and YFP-H148Q (a YFP mutant), the dissociation constant for $Cl^-$ (Kd) is 777 mM and 154 mM, respectively (Wachter and al., J Mol Biol. 2000 Aug 4;301(1):157-71).

Galietta and al (2001) developed a double mutant YFP-H148Q/V163S, which has, at physiological pH (7,35), a Kd of 39mM, and a binding kinetics ($\tau$) of 1900 ms. The other double mutant disclosed is YFP-H148Q/I152L, which has a binding kinetics ($\tau$) of 52 ms and a Kd of 85mM.

Furthermore, the document Kuner and Augustine (2000, Neuron 27:447-459) describes a new ratiometric indicator for chloride ions, termed Clomeleon, based on the coupling of chloride-insensitive CFP with chloride sensitive YFP via a flexible peptide linker for fluorescence-resonance-energy-transfer (FRET), Clomeleon being expressed in cultured neurons by electroporation of plasmide DNA encoding Clomeleon for monitoring of GABA-induced currents.

David-Watine and al (Neuropharmacology. 1999 Jun;38(6):785-92) describes fusion of GFP with alpha subunit (alphaZ1) of the zebrafish Glycine receptor (GlyR).

Another major disadvantage common to all these methods is that they only allow monitoring of an average $[Cl^-]_i$ in all of the volume of the cell. However, for understanding of a number of $Cl^-$-dependent physiological processes, it is important to measure a local $[Cl^-]_i$ in the vicinity of the membrane or around $Cl^-$-selective channels.

So, there is a need for developing a new YFP intracellular anion concentration indicator which has a sensitivity appropriate with intracellular anion concentration combined with a rapid binding kinetics, in particular for measurements of local intracellular anion concentrations.

The present invention resolves this technical problem, which overcomes all the drawbacks of the prior art previously mentioned.

One first aspect of the present invention is a polypeptide which comprises the sequence SEQ ID No. 1 containing at least the two mutations I152L and V163S, or which comprises a sequence analogue to said sequence containing said two mutations.

The terms protein, polypeptide and peptide will be used indifferently in the present description to design an amino acid sequence or their derivatives or analogues containing an amino acid sequence.

The sequence SEQ ID No. 1 is derived from the wild enhanced yellow fluorescent protein (EYFP). This protein is a yellow fluorescent variant of the enhanced green fluorescent protein (EGFP) and has halide binding properties (Wachter & Remington, 1999). Indeed, EYFP fluorescence is pH-sensitive, and its dissociation constant (Kd) can vary on an anion concentration, in particular halide or thiocyanate ion concentrations ([Cl-], [Br-], [I-],....and/or [SCN-]) in a sample, wherein said EYFP is present. With this anion sensitive mechanism of EYFP, it is possible to determine such concentrations in said sample.

The expression "fluorescent protein" refers to any protein capable of fluorescence when excited with appropriate electromagnetic radiation. This includes fluorescent proteins whose amino acid sequences are either natural or engineered.

The term sample in the present application refers to any medium which comprises anions, in particular halide, thiocyanate or nitrate ion concentrations ([Cl-], [Br-], [I-], [SCN-]....and/or $[NO_3$-]) or which is supposed to comprise such anions. It may be, for example but without any limitation, a sample containing cells in culture (e.g., microbiological cultures), a sample containing the cytosol of a cell or the intracellular compartment of a cell.

The polypeptide of the invention, which comprises the sequence SEQ ID No. 1 (or EYFP) with the two mutations I152L and V 163 S, or which comprises a sequence analogue to said sequence (SEQ ID No. 1) containing said two mutations (I152L and V163S), has the following biophysical characteristics, calculated for a given sample ( cf. equation of the example 1) :

- A high affinity for a given anion concentration, in particular a halide, thiocyanate or nitrate ion concentration in said solution, characterized by a low dissociation constant (Kd) ;
- A rapid binding kinetics with a given anion species, in particular a halide, thiocyanate or nitrate ion species, characterized by a low time constant association ($\tau_{assoc}$).

Preferably, the biophysical characteristics Kd and $\tau_{assoc}$ of the polypeptide of the invention are respectively and simultaneously lower than 60 mM and lower than 500ms.

More preferably, said biophysical characteristics Kd and $\tau_{assoc}$ are respectively and simultaneously lower than 50 mM and lower than 100ms.

Even more preferably, said biophysical characteristics Kd and $\tau_{assoc}$ are respectively and simultaneously lower than 45 mM and lower than 100ms.

Most preferably, said biophysical characteristics Kd and $\tau_{assoc}$ are respectively and simultaneously lower than 45 mM and lower than 75 ms.

The expression "analogue sequence" of the sequence SEQ ID No. 1 containing the two mutations I152L and V163S is intended to refer to any fragment or homologue sequence of said sequence containing said two mutations which has biophysical characteristics identical to biophysical characteristics of said sequence containing said two mutations, or which has biophysical characteristics identical to at least 80, 85, 90 or 95 % compared to biophysical characteristics of said sequence containing said two mutations. Such an analogue sequence may have at least 60, 65, 70, 75, 80, 85, 90, 95 or 99 % of identity with sequence SEQ ID No. 1 which comprises at least the two mutations.

The at least two mutations I152L and V163S contained in the sequence SEQ ID No. 1 are substitutions : the mutation at position 152 of SEQ ID No. 1 (or EYFP) leads to the substitution of the amino-acid leucine (L) for the amino-acid isoleucine (I), and the mutation at position 163 of SEQ ID No. 1 (or EYFP) leads to the substitution of the amino-acid serine (S) for the amino-acid valine (V).

The sequence SEQ ID No. 1 containing at least the two mutations I152L and V163S described herein can be produced by any suitable method known in the art. Such methods include constructing a DNA sequence encoding mutant SEQ ID No. 1 sequence and expressing this sequence in a suitable transformed host. This method will produce isolated, mutant SEQ ID No. 1 sequence.

However, the mutant SEQ ID No. 1 sequence may also be produced by chemical synthesis or a combination of chemical synthesis and recombinant DNA technology. In one recombinant method for producing a mutant SEQ ID No. 1 sequence, a DNA sequence is constructed by isolating or synthesizing a DNA sequence encoding SEQ ID No. 1 and then mutagenizing by site-specific mutagenesis.

Directed mutagenesis techniques are well known and have been reviewed by Lather, R. F. and Lecoq, J. P. in Genetic Engineering Academic Press (1983) pp 31-50. Oligonucleotide-directed mutagenesis is specifically reviewed by Smith, M. and Gillam, S. in Genetic Engineering: Principles and Methods, Plenum Press (1981) 3:1-32.

Another method of constructing a DNA sequence encoding the polypeptide or the polypeptidic complex of the present invention would be by chemical synthesis. For example, a mutant DNA sequence encoding a mutant SEQ ID No. 1 sequence may be synthesized by chemical means using an oligonucleotide synthesizer. Such oligonucleotides are designed based on the amino acid sequence of the desired mutant SEQ ID No. 1 sequence, and preferably by selecting those codons that are favored in the host cell in which the recombinant SEQ ID No. 1 sequence will be produced.

Once assembled (by synthesis, site directed mutagenesis or another method), the mutant DNA sequences encoding a mutant SEQ ID No. 1 sequence may be inserted into an expression vector and operatively linked to an expression control sequence appropriate for expression of the mutant SEQ ID No. 1 sequence in a desired host. Proper assembly may be confirmed by nucleotide sequencing, restriction mapping, and expression of a biologically active polypeptide in a suitable host. As is well known in the art, in order to obtain high expression levels of a transfected gene in a host, the gene must be operatively linked to transcriptional and translational expression control sequences that are functional in the chosen expression host.

The choice of expression control sequence and expression vector will depend upon the choice of host. A wide variety of expression host/vector combinations may be employed. Useful expression vectors for eukaryotic hosts, include, for example, vectors comprising expression control sequences from SV40, bovine papilloma virus, adenovirus and cytomegalovirus. Useful expression vectors for bacterial hosts include known bacterial plasmids, such as plasmids from E. coli, including col E1, pCR1, pBR322, pMB9 and their derivatives, wider host range plasmids, such as M13 and filamentous single stranded DNA phages. Preferred E. coli vectors include pL vectors containing the lambda phage pL promoter (U.S. Pat. No. 4,874,702), pET vectors containing the T7 polymerase promoter (Studier et al., Methods in Enzymology 185: 60-89, 1990) and the pSP72 vector (Kaelin et al., supra). Useful expression vectors for yeast cells include the 2.mu. plasmid and derivatives thereof. Useful vectors for insect cells include pVL 941. Useful expression vectors for plant cells include, but are not limited to, cauliflower mosaic virus (CaMV) and tobacco mosaic virus (TMV). In addition, any of a wide variety of expression control sequences may be used in these vectors. Such useful expression control sequences include the expression control sequences associated with structural genes of the foregoing expression vectors. Examples of useful expression control sequences include, for example, the early and late promoters of SV40 or adenovirus, the lac system, the trp system, the TAC or TRC system, the major operator and promoter regions of phage lambda, for example pL, the control regions of fd coat protein, the promoter for 3-phosphoglycerate kinase or other glycolytic enzymes, the promoters of acid phosphatase, e.g., Pho5, the promoters of the yeast .alpha.-mating system and other sequences known to control the expression of genes or prokaryotic or eukaryotic cells and their viruses, and various combinations thereof.

Any suitable host may be used to produce in quantity the polypeptide or the polypeptidic complex of the present invention described herein, including bacteria, fungi (including yeasts), plant, insect, mammal, or other appropriate animal cells or cell lines, as well as transgenic animals or plants. More particularly, these hosts may include well known eukaryotic and prokaryotic hosts, such as strains of E.coli, Pseudomonas, Bacillus, Streptomyces, fungi, yeast, insect cells such as Spodoptera Frugiperda (SF9), and animal cells such as Chinese hamster ovary (CHO) and mouse cells such as NS/O, African green monkey cells such as COS1, COS 7, BSC 1, BSC 40, and BMT 10, and human cells, as well as plant cells in tissue culture.

It should of course be understood that not all vectors and expression control sequences will function equally well to express the polypeptide or the polypeptidic complex of the present invention. Neither will all hosts function equally well with the same expression system. However, one of skill in the art may make a selection among these vectors, expression control systems and hosts without undue experimentation.

Any of the methods known in the art for the insertion of polynucleotide sequences into a vector may be used. See, for example, Sambrook et al., Molecular Cloning: A Laboratory Manual, Cold Spring Harbor Laboratory, Cold Spring Harbor, N.Y. (1989) and Ausubel et al., Current Protocols in Molecular Biology, J. Wiley & Sons, NY (1992), both of which are incorporated herein by reference. Conventional vectors consist of appropriate transcriptional/translational control signals operatively linked to the polynucleotide sequence for producing the polypeptide or the polypeptidic complex of the present invention. Promoters/enhancers may also be used to control expression of polypeptide or the polypeptidic complex of the present invention. Promoter activation may be tissue specific or inducible by a metabolic product or administered substance. Such promoters/enhancers include, but are not limited to, the native E2F promoter, the cytomegalovirus immediate-early promoter/enhancer (Karasuyama et al., J. Exp. Med., 169: 13 (1989)); the human beta-actin promoter (Gunning et al., Proc. Nat. Acad. Sci. USA, 84: 4831 (1987); the glucocorticoid-inducible promoter present in the mouse mammary tumor virus long terminal repeat (MMTV LTR) (Klessig et al., Mol. Cell. Biol., 4: 1354 (1984)); the long terminal repeat sequences of Moloney murine leukemia virus (MuLV LTR) (Weiss et al., RNA Tumor Viruses, Cold Spring Harbor Laboratory, Cold Spring Harbor, N.Y. (1985)); the SV40 early region promoter (Bemoist and Chambon, Nature, 290:304 (1981)); the promoter of the Rous sarcoma virus (RSV) (Yamamoto et al., Cell, 22: 787 (1980)); the herpes simplex virus (HSV) thymidine kinase promoter (Wagner et al., Proc. Nat. Acad. Sci. USA, 78: 1441 (1981)); the adenovirus promoter (Yamada et al., Proc. Nat. Acad. Sci. USA, 82: 3567 (1985)).

Expression vectors compatible with mammalian host cells include, for example, plasmids; avian, murine and human retroviral vectors; adenovirus vectors; herpes viral vectors; and non-replicative pox viruses. In particular, replication-defective recombinant viruses can be generated in packaging cell lines that produce only replication-defective viruses. See Current Protocols in Molecular Biology: Sections 9.10-9.14 (Ausubel et al., eds.), Greene Publishing Associcates, 1989.

Specific viral vectors for use in gene transfer systems are now well established. See for example: Madzak et al., J. Gen . Virol., 73: 1533-36 (1992: papovavirus SV40); Berkner et al., Curr. Top. Microbiol. Immunol., 158: 39-61 (1992: adenovirus); Moss et al., Curr. Top. Microbiol. Immunol., 158: 25-38 (1992: vaccinia virus); Muzyczka, Curr. Top. Microbiol. Immunol., 158: 97-123 (1992: adeno-associated virus); Margulskee, Curr. Top. Microbiol. Immunol., 158: 67-93 (1992: herpes simplex virus (HSV) and Epstein-Barr virus (HBV)); Miller, Curr. Top. Microbiol. Immunol., 158: 1-24 (1992: retrovirus); Brandyopadhyay et al., Mol. Cell. Biol., 4: 749-754 (1984: retrovirus); Miller et al., Nature, 357: 455-450 (1992: retrovirus); Anderson, Science, 256: 808-813 (1992: retrovirus), all of which are incorporated herein by reference.

Another embodiment is a polypeptide of the present invention, wherein said sequence further contains the mutation H148Q.

The sequence SEQ ID No. 1 containing the three mutations I152L, V163S and H148Q described herein can be produced by any suitable method known in the art such as described above. The mutation at position 148 of said wild EYFP leads to the substitution of the amino-acid glutamine (Q) for the amino-acid histidine (H).

The polypeptide of the present invention can further comprise the sequence of a fluorescent protein, said fluorescent protein being capable of exhibiting FRET with the sequence SEQ ID No. 1 containing said mutations or with a sequence analogue to said sequence containing said mutations.

Preferably, the sequence of a fluorescent protein is SEQ ID No. 2 or a sequence having at least 60 % of identity with SEQ ID No. 2.

The obtaining of a polypeptide which comprises two different amino acid sequences according to the present invention is performed using any standard techniques such as previously described.

The sequence SEQ ID No. 2 is derived from the enhanced cyan fluorescent protein (ECFP).

The expression "sequence having at least 60 % of identity with SEQ ID No. 2" refers to any sequence which has at least 60, 65, 70, 75, 80, 85, 90, 95 or 99 % of identity with sequence SEQ ID No. 2.

Another sequence corresponding to a fluorescent protein may be for example, but without any limitation, the sequence of the Enhanced Red Fluorescent Protein (ERFP) (Clontech, Palo Alto, USA).

The polypeptide of the present invention comprising two fluorescent amino acid sequences, such as for example SEQ ID No.2 (ECFP) and SEQ ID No. 1 containing said at least two or three mutations (rEYFP) are used for Fluorescence Resonance Energy Transfer (FRET). Fluorescence resonance energy transfer (FRET) is a distance-dependent interaction between the electronic excited states of two dye molecules in which excitation is transferred from a donor molecule to an acceptor molecule.

Indeed, fluorescent proteins having the proper emission and excitation spectra that are brought into physically close proximity with one another can exhibit FRET. In the polypeptide or the polypeptidic complex of the invention, the two fluorescent amino acid sequences are been chosen such that the excitation spectrum of one of the fluorescent amino acid sequences (the acceptor moiety such as rEYFP) overlaps with the emission spectrum of the excited protein fluorescent amino acid sequence (the donor moiety such as ECFP). The donor moiety such as ECFP is excited by light of appropriate intensity within the rEYFP donor's excitation spectrum. The donor such as ECFP then emits the absorbed energy as fluorescent light. The fluorescent energy it produces is quenched by the acceptor fluorescent protein moiety (rEYFP). FRET can be manifested as a reduction in the intensity of the fluorescent signal from the donor such as ECFP, reduction in the lifetime of its excited state, and reemission of fluorescent light at the longer wavelengths (lower energies) characteristic of the acceptor (rEYFP).

The donor moiety such as ECFP may also be any other fluorescent protein having the above-described properties. It may be, for example, the Red Fluorescent Protein.

Another embodiment is a polypeptide of the present invention, wherein the N-terminal part of the sequence SEQ ID No. 1 containing said mutations is operably linked with the C-terminal part of the sequence of said fluorescent protein.

By the term N-terminal (N-ter) part of one amino acid sequence it is meant the amino-terminal part, or $-N^{+}H_3$ extremity of said sequence. By the C-terminal (C-ter) part of one amino acid sequence it is meant the carboxy-terminal part, or $-COO^{-}$ extremity of said sequence.

"Operably linked" refers to a juxtaposition wherein the components so described are in a relationship permitting them to function in their intended manner.

In the present invention, the N-terminal part of the sequence SEQ ID No. 1 containing said mutations is operably linked with the C-terminal part of the fluorescent protein such as the fluorescent protein of sequence SEQ ID No. 2, which in another words leads to the $ECFP_{-CO-NH-r}EYFP$.

The polypeptide of the present invention can further comprise a peptide linker inserted between the sequence SEQ ID No. 1 containing said mutations and the sequence of said fluorescent protein.

Peptide linkers according to the present invention are any amino acid sequences which are able to influence the FRET technique results, in particular to improve said results obtained with the polypeptide of the invention comprising mutated amino sequence SEQ ID No. 1 (rEYFP) and a fluorescent protein (FP) such as the fluorescent protein of sequence SEQ ID No. 2 (ECFP). Indeed, in the FRET technique, the interaction between said fluorescent amino acid sequences can vary on a (EC)FP-rEYFP distance basis.

Another embodiment is a polypeptide of the present invention, wherein the N-terminal part of said peptide linker is operably linked with the C-terminal part of the sequence of said fluorescent protein, and the C-terminal part of said peptide linker is operably linked with the N-terminal part of the sequence SEQ ID No. 1.

Such a polypeptide comprises the configuration $(EC)FP_{-CO-NH-}linker_{-CO-NH-}$ rEYFP.

Another further embodiment is a polypeptide of the present invention, wherein the sequence of said peptide linker is SEQ ID No. 3, or a sequence having at least 60 % of identity with the sequence SEQ ID No. 3.

By percentage of identity between two nucleic acid or amino acid sequences in the present invention, it is meant a percentage of identical nucleotides or amino acid residues between the two sequences to compare, obtained after the best alignment ; this percentage is purely statistical, and the differences between the two sequences are randomly distributed and all along their length. The best alignment or optimal alignment is the alignment corresponding to the highest percentage of identity between the two sequences to compare, which is calculated such as herein after. The sequence comparisons between two nucleic acid or amino acid sequences are usually performed by comparing these sequences after their optimal alignment, said comparison being performed for one segment or for one "comparison window", to identify and compare local regions of sequence similarity. The optimal alignment of sequences for the comparison can be performed manually or by means of the algorithm of local homology of Smith and Waterman (1981) (Ad. App. Math. 2:482), by means of the algorithm of local homology of Neddleman and Wunsch (1970) (J. Mol. Biol. 48:443), by means of the similarity research method of Pearson and Lipman (1988) (Proc. Natl. Acad. Sci. USA 85: 2444), by means of computer softwares using these algorithms (GAP, BESTFIT, FASTA and TFASTA in the Wisconsin Genetics Software Package, Genetics Computer Group, 575 Science Dr., Madison, WI).

The percentage of identity between two nucleic acid or amino acid sequences is determined by comparing these two aligned sequences in an optimal manner with a "comparison window" in which the region of the nucleic acid or amino acid sequence to compare may comprise additions or deletions with regard the sequence of reference for an optimal alignment between these two sequences. The percentage of identity is calculated by determining the number of positions for which the nucleotide or the amino acid residue is identical between the two sequences, by dividing this number of identical positions by the total number of positions in the "comparison window" and by multiplying the result obtained by 100, to obtain the percentage of identity between these two sequences.

Preferably, the peptide linker sequence of the polypeptide of the present invention has at least 65, 70, 75, 79, 83, 87, 91 or 95 % of identity with the sequence SEQ ID No. 3.

One preferred embodiment is a polypeptide of the present invention, wherein the sequence of said peptide linker is SEQ ID No. 4.

A polypeptide of the invention comprising the sequence SEQ ID No. 1 (EYFP) containing at least said two or three mutations, a fluorescent protein such as for example the fluorescent protein of sequence SEQ ID No. 2 (ECFP) and a peptide linker inserted between the SEQ ID No. 1 (EYFP) containing said mutations and said fluorescent protein will be called in the present application an anionic sensor.

One preferred embodiment is a polypeptide of the present invention having the sequence SEQ ID No. 5, said polypeptide being a preferred example of an anionic sensor.

The polypeptide of the present invention can further comprise a transmembrane anionic channel polypeptide, or a sub-unit or fragment thereof.

By the expression "transmembrane anionic channel polypeptide, or a sub-unit or fragment thereof ", it is meant any polypeptide or sub-unit or fragment thereof which is localised crosswise a lipid bilayer membrane such as the plasma membrane as well as the intracellular and mitochondrial membranes of a cell, and whose tridimensional conformation can form, alone, or with another amino acid sequence such as sub-units, a channel, said channel allowing anionic currents to flow across said lipid bilayer membrane.

One embodiment is the polypeptide of the invention wherein the polypeptide of the invention is inserted in said transmembrane anionic channel polypeptide, or in a sub-unit or a fragment thereof, at the vicinity of its anionic channel on the cytoplasmic domain of said transmembrane anionic channel polypeptide.

The expression "vicinity of the anionic channel on the cytoplasmic domain" of said transmembrane anionic channel polypeptide refers to any location inside the anionic channel or on the cytoplasmic domain of said transmembrane anionic channel polypeptide which allows to monitor dynamic changes in anionic concentrations occurring across the lipid bilayer membrane of a living cell or of a subcellular compartment of a living cell, said transmembrane anionic channel polypeptide being located through said lipid bilayer membrane.

The transmembrane anionic channel polypeptide may be associated with the so-called anionic sensor, allowing to monitor dynamic changes in anion concentrations occurring across the lipid bilayer membrane of a living cell or of a subcellular compartment of a living cell when anion currents are flowing across said lipid bilayer membrane.

Said association is carried out by inserting in the transmembrane anionic channel polypeptide, preferably at the vicinity of its anionic channel on the cytoplasmic domain of said transmembrane anionic channel polypeptide, the polypeptide of the invention.

In a preferred embodiment, the polypeptide of the invention having the sequence SEQ ID No. 5 is inserted in the transmembrane anionic channel polypeptide at the vicinity of its anionic channel on the cytoplasmic domain of said transmembrane anionic channel polypeptide.

Anions which can flow across the lipid bilayer membrane of a living cell or of a subcellular compartment of a living cell according to the present application are any anions which can pass through the anionic channel of said transmembrane anionic channel polypeptide. Halide ions, such as chloride (Cl-), bromide (Br-), iodide (I-) ions, thiocyanate (SCN-) or nitrate ($NO_3$-) ions may be cited, without any limitation.

Preferably, the transmembrane anionic channel polypeptide of the polypeptide of the present invention can be selected from the group consisting of gamma-amino-butyric acid (GABA) receptors, cystic fibrosis transmembrane conductance regulator (CFTR), glycine receptor and acetylcholine receptor, more preferably the glycine receptor.

A most prefered embodiment is a polypeptide of the invention wherein the transmembrane anionic channel polypeptide is the $\alpha$1 sub-unit of a glycine receptor, said $\alpha$1 sub-unit having the sequence SEQ ID No. 6 or a sequence having at least 60 % of identity with SEQ ID No. 6.

By the expression "sequence having at least 60 % of identity with SEQ ID No. 6" it is meant to design any sequence which has at least 60, 65, 70, 75, 80, 85, 90, 95 or 99 % or identity with SEQ ID No. 6.

The glycine receptor is a pentameric ligand-gated transmembrane anionic channel polypeptide composed of $\alpha$ subunits which bind the ligand, and $\beta$ structural subunits. The glycine receptor plays a role in the inhibitory neurotransmission in the central nervous system, mediated by the amino acid glycine. Said amino acid glycine acts as a ligand for the glycine receptor.

In a most preferred embodiment, the polypeptide of the present invention has the sequence SEQ ID No. 7.

The sequence SEQ ID No. 7 is a sequence derived from to the human alpha1 sub-unit of the glycine receptor comprising the anionic sensor containing, from the N-terminal part to the C-terminal part of said anionic sensor, the sequence SEQ ID No. 1 (EYFP) with the three mutations I152L, V163S and H148Q, the peptide linker having the sequence SEQ ID No. 4 and the sequence SEQ ID No. 2 (ECFP).

One second aspect of the present invention is a nucleic acid encoding a polypeptide of the present invention.

The nucleotidic sequence encoding the polypeptide sequence SEQ ID No. 1 (derived from EYFP) may have the sequence SEQ ID No. 8.

The nucleotidic sequence encoding the polypeptide sequence SEQ ID No. 2 (derived from ECFP) may have the sequence SEQ ID No. 9.

The nucleotidic sequence encoding the peptide linker of sequence SEQ ID No. 4 may have the sequence SEQ ID No. 10.

As a preferred embodiment, the nucleic acid encoding the polypeptide of the invention having the sequence SEQ ID No. 5 (anionic sensor) may have the sequence SEQ ID No. 11.

The nucleotidic sequence encoding the polypeptide sequence SEQ ID No. 6 (derived from the α1 sub-unit of a glycine receptor) may have the sequence SEQ ID No. 12.

In a most preferable embodiment, the nucleic acid of the present invention has the sequence SEQ ID No. 13.

One third aspect of the present invention is a vector comprising the nucleic acid of the present invention.

A preferred vector is a vector of the invention comprising the nucleic acid encoding the polypeptide having the sequence SEQ ID No. 5.

More preferably, the vector of the present invention comprises the nucleic acid having the sequence SEQ ID No. 11.

Preferably, the vector of the present invention has been deposited at the CNCM on July 17, 2003 under number I-3070 and comprises the sequence SEQ ID No. 11.
For clonage of said vector, the bacterial strain E. coli DH5α has been used.

More preferably, the vector of the present invention comprises the nucleic acid encoding the polypeptide having the sequence SEQ ID No. 7.

More preferably, the vector of the present invention comprises the nucleic acid having the sequence SEQ ID No. 13.

In a most preferable embodiment, the vector of the present invention has been deposited at the CNCM on July 17, 2003 under number I-3071 and comprises the nucleic acid of sequence SEQ ID No. 11.
For clonage of said vector, the bacterial strain E. coli DH5α has been used.

One fourth aspect of the present invention is a host cell containing the nucleic acid or the vector of the present invention.

A polypeptide of the invention is expressed in a prokaryotic or eukaryotic cell by introducing nucleic acid encoding the polypeptide into a host cell, wherein the nucleic acid is in a form suitable for expression of the polypeptide in the host cell. For example, a recombinant expression vector of the invention, encoding the polypeptide, is introduced into a host cell. Alternatively, nucleic acid encoding the polypeptide which is operatively linked to regulatory sequences (e.g., promoter sequences) but without additional vector sequences can be introduced into a host cell. As used herein, the term "host cell" is intended to include any prokaryotic or eukaryotic cell or cell line so long as the cell or cell line is not incompatible with the protein to be expressed, the selection system chosen or the fermentation system employed. Non-limiting examples of mammalian cell lines which can be used have been previously described.

In addition to cell lines, the invention is applicable to normal cells, such as cells to be modified for gene therapy purposes or embryonic cells modified to create a transgenic or homologous recombinant animal. Examples of cell types of particular interest for gene therapy purposes include hematopoietic stem cells, myoblasts, hepatocytes, lymphocytes, neuronal cells and skin epithelium and airway epithelium. Additionally, for transgenic or homologous recombinant animals, embryonic stem cells and fertilized oocytes can be modified to contain nucleic acid encoding a polypeptide of the invention. Moreover, plant cells can be modified to create transgenic plants.

The invention is broadly applicable and encompasses non-mammalian eukaryotic cells as well, including insect (e.g,. Sp. frugiperda), yeast (e.g., S. cerevisiae, S. pombe, P. pastoris, K. lactis, H. polymorpha; as generally reviewed by Fleer, R. (1992) Current Opinion in Biotechnology 3(5):486-496)), fungal and plant cells. Examples of vectors for expression in yeast S. cerivisae include pYepSec1 (Baldari. et al., (1987) Embo J. 6:229-234), pMFa (Kuijan and Herskowitz, (1982) Cell 30:933-943), pJRY88 (Schultz et al., (1987) Gene 54:113-123), and pYES2 (Invitrogen Corporation, San Diego, Calif.). The polypeptide can be expressed in insect cells using baculovirus expression vectors (e.g., as described in O'Reilly et al. (1992) Baculovirus Expression Vectors: A Laboratory Manual, Stockton Press). Baculovirus vectors available for expression of proteins in cultured insect cells (e.g., SF 9 cells) include the pAc series (Smith e al., (1983) Mol. Cell Biol. 3:2156-2165) and the pVL series (Lucklow, V. A., and Summers, M. D., (1989) Virology 170:31-39).

The number of host cells transformed with a nucleic acid of the invention will depend, at least in part, upon the type of recombinant expression vector used and the type of transfection technique used. Nucleic acid can be introduced into a host cell transiently, or more typically, for long term regulation of gene expression, the nucleic acid is stably integrated into the genome of the host cell or remains as a stable episome in the host cell. Plasmid vectors introduced into mammalian cells are typically integrated into host cell DNA at only a low frequency. In order to identify these integrants, a gene that contains a selectable marker (e.g., drug resistance) is generally introduced into the host cells along with the nucleic acid of interest. Preferred selectable markers include those which confer resistance to certain drugs, such as G418 and hygromycin. Selectable markers can be introduced on a separate plasmid from the nucleic acid of interest or, are introduced on the same plasmid. Host cells transfected with a nucleic acid of the invention (e.g., a recombinant expression vector) and a gene for a selectable marker can be identified by selecting for cells using the selectable marker. For example, if the selectable marker encodes a gene conferring neomycin resistance, host cells which have taken up nucleic acid can be selected with G418. Cells that have incorporated the selectable marker gene will survive, while the other cells die.

Nucleic acids encoding the polypeptide of the invention can be introduced into eukaryotic cells growing in culture in vitro by conventional transfection techniques (e.g., calcium phosphate precipitation, DEAE-dextran transfection, electroporation etc.). Nucleic acid can also be transferred into cells in vivo, for example by application of a delivery mechanism suitable for introduction of nucleic acid into cells in vivo, such as retroviral vectors (see e.g., Ferry, N et al. (1991) Proc. Natl. Acad. Sci. USA 88:8377-8381; and Kay, M. A. et al. (1992) Human Gene Therapy 3:641-647), adenoviral vectors (see e.g., Rosenfeld, M. A. (1992) Cell 68:143-155; and Herz, J. and Gerard, R. D. (1993) Proc. Natl. Acad. Sci. USA 90:2812-2816), receptor-mediated DNA uptake (see e.g., Wu, G. and Wu, C. H. (1988) J. Biol. Chem. 263:14621; Wilson et al. (1992) J. Biol. Chem. 267:963-967; and U.S. Pat. No. 5,166,320), direct injection of DNA (see e.g., Acsadi et al. (1991) Nature 332:815-818; and Wolff et al. (1990) Science 247:1465-1468) or particle bombardment (see e.g., Cheng, L. et al. (1993) Proc. Natl. Acad. Sci. USA 90:4455-4459; and Zelenin, A. V. et al. (1993) FEBS Letters 315:29-32).

Preferably, the host cell of the present invention contains the nucleic acid encoding the polypeptide having the sequence SEQ ID No. 5, or the vector comprising said nucleic acid. More preferably, the host cell of the present invention contains the nucleic acid of sequence SEQ ID No. 11, or the vector comprising said nucleic acid.

Preferably, the host cell of the present invention has been deposited at the CNCM on July 17, 2003 under number I-3070 and comprises the nucleic acid of sequence SEQ ID No. 11.

More preferably, the host cell of the present invention contains the nucleic acid encoding the polypeptide having the sequence SEQ ID No. 7, or the vector comprising said nucleic acid.

More preferably, the host cell of the present invention contains the nucleic acid having the sequence SEQ ID No. 13, or the vector comprising said nucleic acid.

In a most preferable embodiment, the host cell of the present invention has been deposited at the CNCM on July 17, 2003 under number I-3071 and comprises the nucleic acid of sequence SEQ ID No. 11.

One fifth aspect of the present invention is a non-human transgenic animal that comprises a host cell of the present invention.

Nucleic acids or vectors of the present invention encoding one of the polypeptides previously described can be transferred into a fertilized oocyte of a non-human animal to create a transgenic animal which expresses the polypeptide of the invention in one or more cell types. A transgenic animal is an animal having cells that contain a transgene, wherein the transgene was introduced into the animal or an ancestor of the animal at a prenatal, e.g., an embryonic, stage. A transgene is a DNA which is integrated into the genome of a cell from which a transgenic animal develops and which remains in the genome of the mature animal, thereby directing the expression of an encoded gene product in one or more cell types or tissues of the transgenic animal. In one embodiment, the non-human animal is a mouse, although the invention is not limited thereto. In other embodiments, the transgenic animal is a goat, sheep, pig, cow or other domestic farm animal. Such transgenic animals are useful for large scale production of proteins (so called "gene pharming").

A transgenic animal can be created, for example, by introducing a nucleic acid encoding polypeptide (typically linked to appropriate regulatory elements, such as a constitutive or tissue-specific enhancer) into the male pronuclei of a fertilized oocyte, e.g., by microinjection, and allowing the oocyte to develop in a pseudopregnant female foster animal. Intronic sequences and polyadenylation signals can also be included in the transgene to increase the efficiency of expression of the transgene. Methods for generating transgenic animals, particularly animals such as mice, have become conventional in the art and are described, for example, in U.S. Pat. Nos. 4,736,866 and 4,870,009 and Hogan, B. et al., (1986) A Laboratory Manual, Cold Spring Harbor, N.Y., Cold Spring Harbor Laboratory. A transgenic founder animal can be used to breed additional animals carrying the transgene.

It will be appreciated that, in addition to transgenic animals, the nucleic acids or vectors of the present invention encoding one of the polypeptides previously described can be used to create other transgenic organisms, such as transgenic plants. Transgenic plants can be made by conventional techniques known in the art. Accordingly, the invention encompasses non-human transgenic organisms, including animals and plants, that contains cells which express the polypeptide of the invention (i.e., a nucleic acid encoding the polypeptide is incorporated into one or more chromosomes in cells of the transgenic organism).

Another further aspect of the invention is a transgenic plant that comprises a host cell of the present invention.

The transformation of plants can be performed using various appropriated techniques well known by the man skilled in the art, in particular mentioned in the following references : Ed. DUNOD "Physiologie végétale, Tome 2 Developpement" Heller, Esnault and Lance, 2000, Methods of Molecular Biology: Plant gene transfer and expression protocols, Hansen et al., 1999, Recent advances in the transformation of plants, Trends in plant science, vol 4, n°6, 226, S.B.Gelvin, The introduction and expression of transgenes in plants, Current opinion in biotechnology, 1998, 227, Hellens et al., 2000, Trends in plant science, vol 5, n°10, A guide to Agrobacterium binary Ti Vectors, 446, Franken et al., Recombinant proteins from transgenic plants, Current opinion in biotechnology, 8:411-416, Schuler et al., 1998, Insect-resistant transgenic plants, Tibtech, vol 16,168, Michelmore.M, 2000, Genomic approaches to plant disease resistance, Current opinion in biotechnology, 3:125-131.

The plants transformed according to the methods mentioned in the above cited references may be monocotyledones or dicotyledones, in particular maize, wheat, barley, sorghum, potato, pea, soybean, tobacco, canola seed, tomato, sunflower, cotton, rice, bean, cauliflower, broccoli, lettuce, radish, celery, spinach, onion, garlic, carrot, apple, pear, melon, cherry, peach, apricot, raspberry, strawberry, pineapple, avocado, banana, sugarcane.

Preferably, the non-human transgenic animal of the present invention is a mammal. More preferably, the mammal is a mouse.

One sixth aspect of the present invention is a method for the preparation of a transformed cell having a transmembrane anionic channel polypeptide, or a sub-unit or fragment thereof, comprising an anionic sensor expressed at the vicinity of its anionic channel on the cytoplasmic domain of said transmembrane anionic channel polypeptide, said method comprising the following step:

- transforming said cell with the nucleic acid or with the vector of the present invention.

Preferably, the nucleic acid or the vector of the present invention used for transforming said cell encodes the polypeptide having the sequence SEQ ID No. 7.

More preferably, the nucleic acid or the vector of the present invention used for transforming said cell has the sequence SEQ ID No. 13.

In another most preferable embodiment, the vector of the present invention has been deposited at the CNCM on July 17, 2003 under number I-3071.

One further aspect is a method for the detection of anions in a sample, comprising the following steps:

a) contacting said sample with a polypeptide according to the invention;
b) correlating the obtention of a fluorescence signal with the presence of anions in said sample.

One further aspect of the present invention is a method for the determination of anion concentrations in a sample, comprising the following steps :

a) contacting said sample with a polypeptide according to the invention;
b) measuring the obtained fluorescence signal ;
c) correlating said fluorescence signal obtained at step b) with an anion concentration, optionally by comparison with the fluorescence signal of a reference sample having a known anion concentration.

Preferably, the step of contacting said sample with a polypeptide according to the invention of the method for the determination of anion concentrations in a sample is realised with the polypeptide of sequence SEQ ID No.5.

Another aspect is a method for the determination of anion concentrations in the compartment of a living cell, comprising the following steps :

a) transforming said living cell so as to obtain the expression of the polypeptide of the invention in said compartment ;
b) measuring the obtained fluorescence signal ;
c) correlating said fluorescence signal obtained at step b) with an anion concentration, optionally by comparison with the fluorescence signal of a reference living cell compartment having a known anion concentration.

Preferably, in the step of transforming said living cell so as to obtain the expression of the polypeptide according to the invention of the method for the determination of anion concentrations in the compartment of a living cell, the polypeptide according to the invention has the sequence SEQ ID No.5, or has the sequence SEQ ID No. 7.

The method for the determination of anion concentrations in the compartment of a living cell can be performed using various processes known by the man skilled in the art. A preferred process for the determination of anion concentrations in the compartment of a living cell is described in Example 1, paragraph "Determination of chloride concentrations in the intracellular compartment of a living cell".

One another aspect is a method of monitoring pH changes in the compartment of a living cell, comprising the following steps:

a) transforming said living cell with the nucleic acid of anyone of claims 19 to 21 or with the vector of anyone of claims 24 to 27;
b) measuring the fluctuation of the obtained fluorescence signal;
c) correlating said fluctuation of fluorescence signal obtained at step b) with pH changes, optionally by comparison with the fluorescence signal of a reference living cell compartment having a known pH.

One further aspect of the present invention is a method of monitoring dynamic changes in anion concentrations in the compartment of a living cell, comprising the following steps :

a) transforming said living cell with the nucleic acid or with the vector of the invention ;
b) measuring the fluctuation of the obtained fluorescence signal ;
c) correlating said fluctuation of fluorescence signal obtained at step b) with dynamic changes in anion concentrations, optionally by comparison with the fluorescence signal of a reference living cell compartment having a known anion concentration.

Preferably, the step of transforming said living cell with the nucleic acid or with the vector of the invention of the method of monitoring dynamic changes is realised with the nucleic acid or with the vector comprising said nucleic acid encoding the polypeptide of sequence SEQ ID No. 7. Preferably, said nucleic acid encoding the polypeptide of sequence SEQ ID No. 7 has the sequence SEQ ID No. 13.
In another most preferable embodiment, the step of transforming said living cell of the method of monitoring dynamic changes is realised with the vector which has been deposited at the CNCM on July 17, 2003 under number I-3071.
By the expression dynamic changes according to the present invention, it is meant anion fluxes which occur on an anion concentration basis of the solutions localized on the two sides of one cellular or intracellular membrane.
One particular embodiment is the method of monitoring dynamic changes in anion concentrations in the compartment of a living cell of the present invention, wherein said dynamic changes dynamic changes in anion concentrations are monitored on a pH basis, said step b) of measuring the fluctuation of the obtained fluorescence signal being performed at various pH values.
Such pH values are chosen in the range of pH 5 to pH 9. Preferably, pH value is pH6 , pH 7, pH 7,5 or pH 8.
One further aspect of the present invention is a method of selecting a compound capable of modifying anion concentrations in the compartment of a living cell, comprising the following steps :

a) determining the anion concentration using the method for the determination of anion concentrations in the compartment of a living cell of the invention ;
b) contacting said compound with said compartment ;
c) determining the anion concentration using the same method as in step a);
d) selecting the compound when the anion concentration determined at step c) is different from the anion concentration determined at step a).

Another aspect of the present invention is a method of selecting a compound capable of modifying anion concentrations in the compartment of a living cell, comprising the following steps :

a) monitoring the dynamic changes in anion concentration using the method of monitoring dynamic changes in anion concentrations in the compartment of a living cell of the invention ;
b) contacting said compound with said compartment;
c) monitoring the dynamic changes in anion concentrations using the same method as in step a) ;
d) selecting the compound when the dynamic changes determined at step c) are different from the dynamic changes determined at step a).

"A compound capable of modifying anion concentrations" refers to any compound which has the capacity of either increase or decrease anion concentrations in a living cell or in a subcellular compartment of a living cell. A compound capable of increasing or decreasing anion concentrations refers in the present application to a molecule which, when interacting with a transmembrane anionic channel polypeptide, or a transmembrane anionic channel subunit polypeptide, or a fragment thereof, causes the opening or the closing of the anionic channel of said transmembrane anionic channel polypeptide. Compounds which cause the opening of said anionic channel may be called agonists, whereas compounds which cause the closing of said anionic channel may be called antagonists.
One preferred embodiment is one of the methods according to the present invention, wherein said compartment is the intracellular compartment or the subcellular compartment.
One another preferred embodiment is one of the methods according to the present invention, wherein the anion concentrations are halide, thiocyanate (SCN-) or nitrate ($NO_3$-) ion concentrations.
Preferably, the halide ion is selected from the group consisting of chloride, iodure, and bromure ions.
One another embodiment is any method of the present invention, wherein said cell is a eukaryotic cell, preferably a mammal cell or a plant cell.
One further aspect of the present invention is a polypeptide having the sequence SEQ ID No. 4.

Also comprised herein is a nucleic acid encoding the polypeptide having the sequence SEQ ID No. 4. Preferably, said nucleic acid has the sequence SEQ ID No. 10.

One another aspect of the present invention is the use of said polypeptide having the sequence SEQ ID No. 4 as a linker for the operable linkage between two fluorescent proteins involved in FRET.

One further aspect of the present invention is a sensing fluorescent microsphere comprising a polypeptide of the present invention associated with the surface of said microsphere.

Preferably, said polypeptide associated with the surface of said microsphere has the sequence SEQ ID No. 5.

"Sensing fluorescent microsphere" means in the present application any microsphere which has the polypeptide of the invention, preferably the polypeptide of sequence SEQ ID No. 5, associated with its surface and which is able to detect anions in a sample or which is used for analysing variations of said anion concentrations in a sample. Said association may be performed according to various methods well known by the man skilled in the art, such as covalent or affinity binding, or passive adsorption.

Such methods, which will not be detailed in the present description, are largely described in the literature, such as, for example, in the following US patents : US 4,181,636 - US 4,264,766 - US 4,419,444 - US 4,775,619, etc. and in "Latex et diagnostic", Nov. 1996, N° 87,« Le Technoscope », Biofutur ; Duke Scientific Corp. Catalog, Palo-Alto, CA. Technical Note-013A « Reagent Microspheres-Surface properties and Conjugation Methods ».

Suitable microspheres have commonly a diameter of 1 to 1000 nm and are composed of various polymers such as polystyrene, maleic acid-styrene, methacrylic acid-styrene, acrylamide-styrene, polymethylmethacrylate, vinylbenzenechloride-styrene, glycidylmethacrylate-styrene, divinylbenzene, polyvinylpyridine, butadiene-styrene copolymers, butadiene-acrylonitrile copolymers, polyesters, vinyl ester acrylate-acetate copolymers, vinyl ester chlorure-acrylate copolymers, polyethers, polyolefines, polyalkylene oxides, polyamides, polyurethanes. Those microspheres are provided by various firms such as Spherotech (Libertyville, US), Polysciences (Warrington, US), Merck Eurolab SA (Fontenay-sous-Bois, FR), Duke Scientific (Palo Alto, US), Seradyn (Indianapolis, US), Dynal Biotech (Oslo, NO), Bang's Labs (Fishers, US), Polymer Laboratories Ltd (Church Stretton, UK).

Furthermore is comprised in the present invention the use of a sensing fluorescent microsphere of the invention for the detection of anions in a sample, or for the variation analysis of anion concentrations in a sample.

Another further aspect of the invention is a kit for the detection and/or for the determination of the concentration of anions in a sample which comprises the sensing fluorescent microsphere of the present invention.

Kits typically comprise two or more components necessary for performing said detection or for performing a diagnostic assay. Components may be compounds, reagents, containers and/or equipment. For example, one container within a kit may contain a microsphere colloid wherein said microspheres have the polypeptide of the invention, preferably the polypeptide of sequence SEQ ID No. 5, associated with their surface. Such container within a kit may also contain a polypeptide, a nucleic acid, a vector, or a host cell of the present invention. One or more additional containers may enclose elements, such as reagents or buffers, to be used in the assay.

Another aspect of the invention is a kit for monitoring pH changes in a sample, which comprises the sensing fluorescent microsphere of the invention.

One additional aspect of the invention is a kit for monitoring dynamic changes in anion concentrations in a living cell or in a subcellular compartment of a living cell, which comprises a polypeptide, a nucleic acid, a vector, or a host cell of the present invention.

More preferably, the kit for monitoring said dynamic changes of the invention may further comprise a polypeptide having the sequence SEQ ID No. 5 or a polypeptide having the sequence SEQ ID No. 7.

Said kit may also comprise a nucleic acid encoding said polypeptide having the sequence SEQ ID No. 5 or the sequence SEQ ID No. 7, or a vector comprising said nucleic acid, or a host cell containing said nucleic acid or said vector.

More preferably, the kit for monitoring said dynamic changes of the invention comprises the nucleic acid of sequence SEQ ID No. 11 or the nucleic acid of sequence SEQ ID No. 13. Said kit may further comprise a vector comprising said nucleic acid.

In a most preferred embodiment, the kit for monitoring said dynamic changes of the invention comprises the vector or the host cell which has been deposited at the CNCM on July 17, 2003 under number I-3070.

In a most preferred embodiment, the kit for monitoring said dynamic changes of the invention comprises the vector or the host cell which has been deposited at the CNCM on July 17, 2003 under number I-3071.

One additional aspect of the invention is a kit for the diagnosis of a condition involving a transmembrane anionic channel, which comprises the polypeptide of the invention, or the nucleic acid of the invention, the vector of the invention, or the host cell of the invention.

Preferably, the kit for the diagnosis of a condition involving a transmembrane anionic channel of the invention comprises a polypeptide having the sequence SEQ ID No. 7.

Said kit may also comprise a nucleic acid encoding said polypeptide having the sequence SEQ ID No. 7, or a vector comprising said nucleic acid, or a host cell containing said nucleic acid or said vector.

More preferably, the kit for the diagnosis of a condition involving a transmembrane anionic channel of the invention comprises the nucleic acid of sequence SEQ ID No. 13. Said kit may further comprise a vector comprising said nucleic acid.

In a most preferred embodiment, the kit for the diagnosis of a condition involving a transmembrane anionic channel of the invention comprises the vector or the host cell which has been deposited at the CNCM on July 17, 2003 under number I-3071.

One preferred embodiment is the kit for the diagnosis of a condition involving a transmembrane anionic channel of the invention, wherein said condition is selected from the group consisting of cystic fibrosis, myotonia congenita, congenital chloride diarrhoea, inherited hypercalciuric nephrolithiasis, Bartter's and Gitelman's and hyperekplexia/startle disease.

One further aspect of the invention is a polypeptidic complex comprising a transmembrane ionic channel or a sub-unit or fragment thereof, and a polypeptidic biosensor which is capable to emit a specific signal correlated to an ion concentration.

Polypeptidic biosensors are well known by the man skilled in the art, and are described for example, but without any limitation, in the following documents : Reiff and al, 2002 Nov 1 ;22(21) :9399-409 ; Diegelmann and al, 2002 sept-Oct ;34(1-2): 95-8 ; Isshikiand and al, 2002 Nov 8 ;277(45) :43389-98.

A preferred embodiment is a polypeptidic complex according to the present invention, wherein the polypeptidic biosensor is a polypeptidic fluorescence biosensor and the specific signal is a fluorescence signal. Preferably, said polypeptidic fluorescence biosensor comprises two fluorescent proteins and is capable of exhibiting FRET.

A more preferred embodiment is a polypeptidic complex according to the present invention, wherein one the two fluorescent proteins is EYFP. Preferably, the EYFP protein comprises at least one mutation, said mutation being selected in the group consisting of I152L, V163S and H148Q. More preferably, the EYFP protein comprises two mutations selected in said group.

A most preferred embodiment is a polypeptidic complex according to the present invention, wherein said polypeptidic complex comprises the polypeptide of the present invention.

One further aspect is a method for the preparation of a transformed cell having a transmembrane ionic channel polypeptide, or a sub-unit or fragment thereof, comprising a ionic sensor expressed at the vicinity of its ionic channel on the cytoplasmic domain of said transmembrane ionic channel polypeptide, said method comprising the following step :

- transforming said cell so as to obtain the expression of the polypeptidic complex of the present invention in said cell.

Another aspect is a method for the determination of ion concentrations in the compartment of a living cell, comprising the following steps :

a) transforming said living cell so as to obtain the expression of the polypeptidic complex of the present invention in said compartment;
b) measuring the obtained fluorescence signal ;
c) correlating said fluorescence signal obtained at step b) with an ion concentration, optionally by comparison with the fluorescence signal of a reference living cell compartment having a known ion concentration.

Another aspect is a method of monitoring dynamic changes in ion concentrations in the compartment of a living cell, comprising the following steps :

a) transforming said living cell so as to obtain the expression of the polypeptidic complex of the present invention in said compartment;
b) measuring the fluctuation of the obtained fluorescence signal ;
c) correlating said fluctuation of fluorescence signal obtained at step b) with dynamic changes in ion concentrations, optionally by comparison with the fluorescence signal of a reference living cell compartment having a known ion concentration.

Another aspect is a method of selecting a compound capable of modifying ion concentrations in the compartment of a living cell, comprising the following steps :

a) determining the ion concentration using the method for the determination of ion concentrations in the compartment of a living cell of the invention ;
b) contacting said compound with said compartment;
c) determining the ion concentration using the same method as in step a);

d) selecting the compound when the ion concentration determined at step c) is different from the ion concentration determined at step a).

Another aspect is a method of selecting a compound capable of modifying ion concentrations in the compartment of a living cell, comprising the following steps :

a) monitoring the dynamic changes in ion concentrations using the method of monitoring dynamic changes in ion concentrations in the compartment of a living cell according to the invention ;
b) contacting said compound with said compartment;
c) monitoring the dynamic changes in ion concentrations using the same method as in step a) ;
d) selecting the compound when the dynamic changes determined at step c) are different from the dynamic changes determined at step a).

One further aspect is a sensing fluorescent microsphere comprising a polypeptidic complex of the present invention associated with the surface of said microsphere.
One further aspect is a kit for monitoring dynamic changes in ion concentrations in a living cell or in a subcellular compartment of a living cell, which comprises the polypeptidic complex of the present invention.
One further aspect is a kit for the diagnosis of a condition involving a transmembrane ionic channel, which comprises the polypeptidic complex of the present invention.
The purpose of the legends of the figures and of the examples below is to illustrate the invention. It doesn't limit the scope of the claimed invention.

LEGENDS OF THE FIGURES

**Figure 1 : Chemical structure of CFP/YFP-based $Cl^-$-sensor.** Linear representation of the CFP/YFP fusion construct.
This new hybrid protein corresponds to the fusion of ECFP with a Cl-sensitized mutant of EYFP using a linker allowing FRET.
**Figure 2: Excitation spectra of $Cl^-$-sensor in the presence of two different chloride concentrations (140 mM and 7 mM).** HEK-293 cells were transfected with cDNA of CFP/YFP-based $Cl^-$-sensor 24 hours before the monitoring. Cells were excited at different wavelengths using TILL monochromator. Fluorescence signals were recorded at 515 nM. Note that lowering of chloride results in decreasing of emission intensity. However, this effect is much more pronounced at excitation 480 nm then at 430 nm.
**Figure 3: Monitoring of glycine-mediated changes of intracellular chloride concentration in HEK-293 cells co-expressing CFP/YFP-based $Cl^-$-sensor and alpha subunit of human glycine receptor (GlyR) :**

HEK-293 cells were transfected 24 hours before the monitoring.
**A-C**, Images of HEK 293 cells before (A), during (B) and after (C) application of solution with low (7mM) chloride concentration and 1 mM glycine.
**D**, Changes in ratio of fluorescence emission at excitation 430/480 nm. Emission signals were recorded at 515 nm. Note increase of 430/480 ratio at lowering of chloride.
Glycine 1 mM was applied to increase permeability of $Cl^-$-selective GlyR channels expressed in HEK cells.

**Figure 4: Functional YFP-GlyR construct with YFP at cytoplasmic loop. 4A**, Traces of ionic currents measured from HEK 293 cell transfected with d type human GlyR alphal subunit or with GlyR-YFP construct. glycine (30µM) was applied during the time indicated by black application bars. Membrane potential was -50 mV.
**4B**, Dose-response curves for two cells expressing either the wild type GlyR or GlyR-YFP construct. Note similar maximal currents and EC50s for both receptors.
**Figure 5 : YFP fused to cytoplasmic loop of human GlyR exhibit preferable membrane distribution :**

Fluorescence distribution in HEK 293 cells expressing either wild-type GFP (left) or GlyR-YFP (right). cDNA encoding YFP was incorporated into large cytoplasmic domain of human alpha GlyR subunit. Fluorescence images are obtained on confocal microscope with a z-plane depth 0.4 µm. Note the preferable membrane distribution of GlyR-YFP fluorescence.

**Figure 6 : Simultaneous monitoring of GlyR currents and Cl-dependent fluorescence using a**

**BioSensor-Cl :**

HEK-293 cells were transfected with BioSensor-GlyR cDNA 24 hours before the monitoring. Different concentrations of glycine were applied using a fast perfusion system (see scheme in insert).
Whole-cell recording at holding potential -60 mV. Application of glycine (300 µM or 1 mM) causes changes of Cl- induced current (top traces) and consequent decrease of fluorescence intensity (bottom traces). Note increase of ionic currents amplitude and fluorescence intensity at elevation glycine concentration.

**Figure 7 : Non-invasive monitoring of GlyR activation using a BioSensor- GlyR.**
HEK-293 cells were transfected with BioSensor-GlyR cDNA 24 hours before the monitoring.
**Top trace :** fluorescence intensity at excitation 430 nm from the cell recorded in whole-cell patch-clamp configuration at holding potential -60 mV.
**Bottom trace** : non-invasive monitoring of fluorescence intensity from neighbouring cell.
Note that application of glycine vertical lines causes similar changes of fluorescence in both cells.

EXEMPLES

**EXEMPLE 1 : MATERIAL AND METHODS**

**Determination of halide or thiocyanate sensitivities of YFP mutant proteins of the invention :**

See *Galietta and al, FEBS Letters 499 (2001) 220-224.*

Dissociations constants (Kd) were determined from fluorescence (F) titrations using the following equation :

$$F = a[X-]/(Kd + [X-]) + b$$

Where [X-] is halide or thiocyanate concentration and 1-b/a is the fractional decrease in fluorescence at high [X-].

**Kinetic measurements of associations and dissociations between YFP mutant proteins and halide or thiocyanate ions (X-) :**

Kinetic measurements of YFP - X- association and dissociation were done on a Hi-Tech SF51 stopped-flow apparatus using 500 ± 10 nm excitation filter and 530 nm long-pass emission filter. The time course of mutant YFP fluorescence (in 50 mM Na phosphate buffer, pH 7,35) was measured in response to rapid changes in solution [X-] from 0 to 40 mM and from 40 to 20 mM.

**Expression of GlyRs and halide sensor :**

Transfections of the pMT3-αZ1 and pMT3-αZ1-GFP were carried out in human kidney derived cell lines (HEK293 and BOSC23) using a calcium phosphate transfection procedure.
Whole-cell patch-clamp recordings were conducted 24-48h after transfection using EPC-9 amplifier (HEKA Elektronik, Germany) at room temperature (~25 °C). Cells were bathed in solution containing (mM): NaCl 140, $CaCl_2$ 2, KCl 2.8, $MgCl_2$ 2, HEPES/NaOH 10, glucose 10; pH 7.3. Patch pipette solution contained (mM): CsCl 140, MgATP 2, HEPES/CsOH 10, BAPTA 5; pH 7.3. Series resistance (5-15 MΩ) was always compensated on 85-90%. Agonists were applied using a fast perfusion system (SF 77A, Warner, USA).
Complete solution exchange occurred in ~4 ms, as measured by open electrode controls (1/10 NaCl).
Conventional expression of GlyRs in *Xenopus* oocytes and microelectrode voltage-clamp recordings were used [David-Watine et al., 1998].
For quantitative estimations of agonist and antagonist actions, dose-response relationships were fitted with the equations:

$$I = I_{max}/(1 + (EC_{50}/[C])^n) \text{ and } I = I_0(1 - [A]^n/([A]^n + (IC_{50})^n)$$

where **I** is the amplitude of ionic current induced by the agonist at concentration **[C],** $\mathbf{I_{max}}$ is the maximum re-

sponse, $\mathbf{I_0}$ is the glycine-induced current in the absence of antagonist, $\mathbf{EC_{50}}$ is the concentration for which a half-maximum response is induced, $\mathbf{IC_{50}}$ is the concentration of antagonist at which the agonist-induced current is inhibited by 50% and **n** is the Hill coefficient.

**Digital-imaging fluorescence microscopy.**

Fluorescence images were acquired using a customized digital imaging microscope. Various wavelength excitation of cells was achieved using a 1nm bandwidth polychromatic light selector equipped with a 100W xenon lamp (*PolychromeII, TILL Electronics,* Germany). The light intensity was attenuated to ~1-5% of the primary light source intensity using neutral density filters. A dichroic mirror (*XF04 Omega Optics,* USA) was used to deflect light onto the samples, and to pass measured emission light (>515nm). Fluorescence was visualized using an upright microscope (*Axioskop, Zeiss,* Germany) equipped with an infinity corrected 60x water immersion objective (n.a.=0.9; *LumPlanFL, Olympus,* USA), whose focus was controlled using a high speed servo modulated piezo-electric drive apparatus (minimum step 0.1µm; *Physik Instrumente,* Germany). The theoretical point resolution and focal depth of the objective were calculated (using Raleigh's criterion for resolving power, and according to Taylor & Salmon [14] to be 0.34µm on the x-y axis, and 0.71µm on the z-axis. A 4x tv relay optic passed fluorescent emission light to a 12bit charge coupled device digital camera system equipped with image intensifier (*PentaMax512EEV-GENIV, Princeton Instruments,* USA). Images were acquired on a computer *via* a DMA serial transfer. All peripheral hardware control, image acquisition, and image processing were achieved using customized routines inside *METAFluo* software (*International Imaging, USA*).

**Determination of chloride concentrations in the intracellular compartment of a living cell:**

The chloride sensor was expressed in human kidney derived cell lines (HEK293 and BOSC23) using a calcium phosphate transfection procedure.

Measurements of fluorescence are likely to reflect the average [Cl-] in the intracellular compartment.

The ratio (R) of fluorescence emission of the EYFP acceptor to that of the ECFP donor is $R=F_{527}/F_{485}$.

To calibrate the fluorescence of chloride sensor within these cells, R is measured while using a patch pipette to manipulate the cytoplasmic concentration of anions. A nominally Cl- free solution containing 150 mM gluconate is used to determine the maximal emission ratio for the chloride sensor (Rmax), whereas a solution containing a saturating concentration (150mM) of Cl- defines the minimal emission ratio (Rmin). The relationship between R and intermediates values of [Cl-] is then determined. This relationship could be described as :

$$[Cl^-] = K'd\ (Rmax - R)/(R - Rmin),$$

with Rmax = 2,39, Rmin = 0,49 and K'd = 87mM.
this calibration curve can then be used to convert R values measured in intracellular compartment of living cells into [Cl-].

**EXEMPLE 2 : Monitoring of chloride dynamics in HEK cells expressing a fluorescent halide sensor.**

Functional properties of ChlorSensor were analyzed on HEK cells co-expressing homomeric GlyR channels. Transiently transfected cells were brightly fluorescent with a cytoplasmic and nuclear distribution of ChlorSensor. Its excitation spectra showed a maximal peak at 460-465 nm with a clear minimum at 480-485 nm (Fig. 2).

For rapid changes of intracellular chloride concentration, a solution containing low Cl- concentration (7 mM) and 2 mM glycine (for controlled changes of Cl- permeability by GlyR channels activation) was applied to surface of fluorescent cells. This caused a strong decrease of fluorescence intensity, with particularly pronounced effect at 480-485 nm (Fig. 2). As a result the ratio of fluorescence (330/ 380 nm) strongly increased (Fig. 3). Application of low Cl- solution without glycine had no significant effect at this time scale of recording. Effect was also absent when choline was applied instead of glycine and when glycine was applied "high Cl-" solution. At long-lasting (several minutes) application of "low-Cl- " solution, reversible decrease of fluorescdence and corresponding increase of 330/ 380 nm ration were observed. These results demonstrate that, rapid fluorescence changes are due to Cl- concentration changes at GlyR activation and our construct could be used as a sensitive indicator of Cl- concentration changes. Moreover, this construc can be used for monitoring Cl- concentration in ratiometric experiments.

The next step was devoted to obtaining a construct of GlyR fused with chloride sensor and capable to sense changes of Cl concentration in the close vicinity of GlyR channel.

**EXEMPLE 3 : Obtaining functional glycine receptor channel fused with YFP at cytoplasmic domain.**

Previously, we constructed a functional GlyR fused with GFP and used it to study GlyR distribution in living cells (David-Watine et al., 1999). In this construct, GFP was fused to the extracellular domain of GlyR. For monitoring changes of intracellular chloride concentration caused by activation anion-selective channels, it is nesessary to position halide sensor in the vicinity of these channels on the cytoplasmic side of the membrane.

To achieve this aim, we primary developed a strategy for incorporation of YFP in the cytoplasmic domain of GlyR. To this end, a library of random mutants of the GlyR cytoplasmic loop was generated using insertional mutagenesis. From the resulting mutants, several candidates were selected for electrophysiological analysis. cDNAs of each GlyR-YFP construct were transfected into HEK 293 cells and studied using the whole-cell recording technique. This strategy allowed selection of functional clone, which exhibited properties similar to the wild type GlyR. Analysis of concentration-dependencies showed that $EC_{50}$ and maximal currents evoked by saturated concentrations of glycine are similar, while at long application responses of GlyR-YFP showed faster desensitization (Fig. 4).

Confocal imaging revealed membrane and intracellular (Golgi apparatus and endoplasmic reticulum) distribution of GlyR-YFP fluorescence (Fig.5). These results demonstrate that the fusion of YFP to the cytoplasmic domain of GlyR gives a functional channel. For obtaining construct allowing high-resolution fluorescent monitoring of intracellular Cl- concentration we substituted YFP in the cytoplasmic domain of functional GlyR-YFP by Chloride Sensor. This construct we called BioSensor- GlyR.

**EXEMPLE 4 : Non-invasive monitoring of GlyR activity using halide sensor.**

To analyze properties of BioSensor- GlyR,we expressed it in HEK 293 cells. Confocal images revealed that in contrast with the homogeneous cytoplasmic distribution of ChlorSensor, distribution of BioSensor- GlyR was nonhomogeneous with primary presence in a plasma membrane. In addition, fluorescence was excluded from nucleus and it was also present in Golgi apparatus, as well as in some parts of cytoplasm exhibiting distinct bright spots.

To test the suitability of BioSensor- GlyR for monitoring of GlyR activity, we analyzed properties of fluorescent intensity at application glycine to surface of fluorescent cells using a rapid perfusion application system. At whole-cell patch-clamp recordings, glycine (2 mM) was applied in "low Cl" solution containing 7 mM of chloride). Fig. 6 shows simultaneous monitoring of ionic currents and fluorescent emission signals at 430 nm excitation wavelength. Fluorescence was characterized by following properties. First, it intensity increased with elevation of glycine concentration. Second, increase in fluorescent signal intensity was proportional to the charge passing through the membrane at GlyR channels activation. Actually, fluorescence changes were higher in comparison with currents, presumably reflecting involvement of intracellularly situated BioSensor- GlyRs. Third, kinetics of fluorescent signals was slower in comparison with ionic currents. This reflects relatively slow changes in Cl- concentration in the whole volume of the cell. At short pulses of glycine (50 ms) the rise time and recovery fluorescent signals were much faster (hundreds milliseconds). Finally, application of glycine-free solution did not change fluorescence, demonstrating that observed changes reflect activation of GlyR channels.

Using of BioSensor- GlyR allows non-invasive monitoring of GlyR activity. This was demonstrated at simultaneous monitoring from two fluorescent cells. Only one of them was patch-clamped (Fig. 7, top trace). Both cells exhibited fluorescent changes upon glycine application. In patch-clamped cells recovery of fluorescent signal was waster, presumably due to more rapid recovery of Cl- concentration.

Altogether these results demonstrate that BioSensor- GlyR is a highly sensitive tool for non-invasive monitoring of GlyR activity in living cells.

**EXEMPLE 5 : CONCLUSION :**

Here is disclosed a novel halide sensor allowing non-invasive monitoring of chloride dynamics in living cells. This construction has three main advantages over previous ones:

**1. Improved sensitivity to $Cl^-$ and rapid time resolution..**

Previously proposed $Cl^-$indicators based on YFP had relatively low sensitivity to $Cl^-$. Thus, at physiological pH (7.5), for YFP and YFP-H148Q (mutant with enhanced $Cl^-$ sensitivity), the binding apparent affinity constant for $Cl^-$ (Kd) is, 777 mM and 154 mM, respectively (Wachter et al., 2000). The low sensitivity to $Cl^-$ highly limits the practical value of this original YFP $Cl^-$ indicator. For instance, in majority of cells in animals and plants intracellular [$Cl^-$] is less than 40 mM; thus using these proteins for biological and medical purposes is restricted.

Double mutant YFP-H148Q/V163S has much higher $Cl^-$ sensitivity (Kd=39mM), however, it exhibits very slow binding kinetics ($\tau$=1900 ms, Galietta et al., 2001), which strongly limits the time resolution when using this mutant. In contrast the other double mutant, YFP-H148Q/I152L has rapid binding kinetics (τ=52 ms, Galietta et al., 2001), however, its sensitivity to $Cl^-$ is relatively low (Kd=85mM).

The present triple YFP mutant preserves advantages of a relatively high sensitivity to $Cl^-$ and rapid binding kinetics and thus exhibits improved properties allowing a high range of $Cl^-$-sensor application.

**2. Possibility of ratiometric analysis at dual-wavelength excitation.**

A significant limitation in the use of a $Cl^-$-indicators has been a lack of a $Cl^-$- dependent change in spectral shape, which precludes ratiometric measurements. Recently synthesis of series of dual-wavelength $Cl^-$-sensitive fluorescent indicators has been achieved (Jayaraman et al., 1999). However, specific conjugation of chemical compounds with proteins is difficult, which highly limit their use for specific targeting in cellular compartments.

The present CFP/YFP-fused construct allows fluorescence resonance energy transfer (FRET) between CFP and triple mutated YFP and this molecule can be used as a ratiometric indicator.

Figure 3 illustrates that excitation spectra of our $Cl^-$-sensor obtained at high (140 mM) and low (7 mM) [$Cl^-$] show remarkable differences in changes of emission intensity at different excitation wavelengths. Thus, changes in the emission intensity at excitation, 430nm and 480 nm were, respectively 29% and 77%. This feature enables highly improved sensitivity of $Cl^-$-sensor by ratiometric analysis at dual-wavelength excitation (for instance, 430/480 nm).

**3. Possibility of genetically targeted expression.**

In many cases changes of $Cl^-$ take place in local environments. For instance in biological preparations the main changes in $Cl^-$ concentration take place in close vicinity to the cellular plasma membrane. Previously proposed $Cl^-$ indicators do not allow direct monitoring of these near-membrane $Cl^-$ fluxes. These fluorescent dyes are homogeneously distributed in the cytoplasm and thus allow detection of only a mean changes of $Cl^-$ concentration in the whole volume of cells.

Thus, CFP/YFP-based $Cl^-$-sensor provides a possibility of genetically targeted expression with transmembrane proteins. The inventors introduced $Cl^-$-sensor in the cytoplasmic domain of chloride-permeable glycine receptor (GlyR) protein. This permits monitoring of changes in $Cl^-$ concentration in the local surrounding of GlyR channels. A similar strategy can be used for other anion-selective channels. Moreover, incorporation of this construct into mouse genome opens the possibility of non-invasive monitoring of neuronal activity by recording fluorescence changes in synapses containing $Cl^-$-selective channels.

**The practical applications of this new fluorescent halide-sensor concern in general.**

The analysis of the physiological and physiopathological function of chloride and other anions ($I^-$, $Br^-$, $SCN^-$) in cells, tissues, animals, people and in non-living matter.

**And more specifically:**

1. Monitoring of [$Cl^-$] in biomembrane vesicles and liposomes (see problem in .Bae & Verkman, 1990; Quigley et al., 2002)
2. Analysis of anion permeability in cells containing transmembrane $Cl^-$- selective channels, $Cl^-$-transporters or exchanges (see problem in Kawano et al., 1999).
3. Analysis of intracellular [$Cl^-$] in cells with disorders of $Cl^-$-selective channels $Cl^-$-transporters or exchanges (see problems in Archkoph, 2000).
4. Development of optical chloride sensing fluorescent microspheres (see problem in Ceresa et al., 2003).
5. Development of various types of pH and $Cl^-$ probes (reusable peroral, transnasal, and endoscopic) (see problem in Kneen, 1998; Yakovlev, 2001). YFP-based sensors change rapidly and reversibly their fluorescent emission in response to changes in the concentration of chloride and also of some small anions such as halides ($I^-$, $Br^-$ ). Fluorescence is suppressed due to protonation of the chromophore upon anion binding, with the stronger level of interaction at low pH (Kuner & Augustin, 2000). Thus, the present $Cl^-$-sensor can be also used for pH monitoring.
6. Non-invasive monitoring of intracellular chloride in cells during development (see problem in Cherubini et al., 1991; Ben-Ari, 2002).
7. Non-invasive visualization activity of synapses containing $Cl^-$-selective ionic channels: GABAergic and/or glutamatergic synapses in brain of vertebrates; glutamatergic and cholinergic synapses in nervous system of invertebrates.

8. Non-invasive visualization activity of cells containing $Cl^-$-selective channels or $Cl^-$ transporters, or $Cl^-/HCO3^-$ exchangers, or other systems performing transmembrane translocation of chloride.
9. Iodine deficiency is the main cause for potentially preventable mental retardation in childhood (Wu et al., 2002). Probes based on $Cl^-$-sensor can be used for monitoring of free iodine content in diseased with iodine balance disorders.
10. Development of rapid quantitative screening of compounds modifying $Cl^-$ transport in cells with $Cl^-$ balance disorders: cystic fibrosis (Kerem et al., 1989), *myotonia congenita* (Koch et al., 1992), inherited hypercalciuric nephrolithiasis (Lloyd et al., 1996), Barter syndrome (Simon et al., 1996), epilepsy (Treiman, 2001), hyperkplexia/ startle disease (Shiang et al., 1993; Bregestovski, 2002) and pulmonary oedema (Matthay, 2002).
11. Monitoring of $Cl^-$ in plants.

$Cl^-$ is a key element in the regulation of cell water content, conductance and also intracellular pressure in animals and plants (Acher, 2002; Xiong et al., 2002). Use of $Cl^-$-sensor allows measurement of changes in $[Cl^-]$ in plant cells during water rises under different environmental conditions. $Cl^-$-sensor can be used for monitoring the $[Cl^-]$ in plant cells for:

(i) estimation of time course of the water rise kinetics in plants (Moya et al., 2003);
(ii) analysis of stress signal transduction in plants (Zhu, 2002);
(iii) genetic engineering of plants with improved salt tolerance (Serrano, 1996)

## Claims

1. A polypeptide which comprises the sequence SEQ ID No. 1 containing at least the two mutations I152L and V163S, or which comprises a sequence analogue to said sequence containing said two mutations.

2. The polypeptide of claim 1, wherein said sequence further contains the mutation H148Q.

3. The polypeptide of claim 1 or 2 which further comprises the sequence of a fluorescent protein, said fluorescent protein being capable of exhibiting FRET with the sequence SEQ ID No. 1 containing said mutations or with a sequence analogue to said sequence containing said mutations.

4. The polypeptide of claim 3 wherein the sequence of a fluorescent protein is SEQ ID No. 2 or a sequence having at least 60 % of identity with SEQ ID No. 2.

5. The polypeptide of claim 4, wherein the N-terminal part of the sequence SEQ ID No. 1 containing said mutations is operably linked with the C-terminal part of the sequence of said fluorescent protein.

6. The polypeptide of anyone of claims 3 to 5, which further comprises a peptide linker inserted between the sequence SEQ ID No. 1 containing said mutations and the sequence of said fluorescent protein.

7. The polypeptide of claim 6, wherein the N-terminal part of said peptide linker is operably linked with the C-terminal part of the sequence of said fluorescent protein, and the C-terminal part of said peptide linker is operably linked with the N-terminal part of the sequence SEQ ID No. 1.

8. The polypeptide of claim 6 or 7, wherein the sequence of said peptide linker is SEQ ID No. 3, or a sequence having at least 60 % of identity with the sequence SEQ ID No. 3.

9. The polypeptide of anyone of claims 6 to 8, wherein the sequence of said peptide linker is SEQ ID No. 4.

10. The polypeptide of claim 9, having the sequence SEQ ID No. 5.

11. The polypeptide of anyone of claims 1 to 10, which further comprises a transmembrane anionic channel polypeptide, or a sub-unit or fragment thereof.

**12.** The polypeptide of claim 11, wherein the polypeptide of anyone of claims 1 to 10 is inserted in said transmembrane anionic channel polypeptide, or in a sub-unit or a fragment thereof, at the vicinity of its anionic channel on the cytoplasmic domain of said transmembrane anionic channel polypeptide.

**13.** The polypeptide of claim 10 or 11, wherein said transmembrane anionic channel polypeptide is selected from the group consisting of gamma-amino-butyric acid (GABA) receptors, cystic fibrosis transmembrane conductance regulator (CFTR), acetylcholine receptor and glycine receptor.

**14.** The polypeptide of anyone of claims 11 to 13, wherein the transmembrane anionic channel polypeptide is the $\alpha 1$ sub-unit of a glycine receptor, said $\alpha 1$ sub-unit having the sequence SEQ ID No. 6 or a sequence having at least 60 % of identity with SEQ ID No. 6.

**15.** The polypeptide of claim 14, having the sequence SEQ ID No. 7.

**16.** A nucleic acid encoding the polypeptide of anyone of claims 1 to 15.

**17.** A nucleic acid encoding the polypeptide of claim 10.

**18.** The nucleic acid of claim 17, having the sequence SEQ ID No. 11.

**19.** A nucleic acid encoding the polypeptide of anyone of claims 11 to 15.

**20.** A nucleic acid encoding the polypeptide of claim 15.

**21.** The nucleic acid of claim 20, having the sequence SEQ ID No. 13.

**22.** A vector comprising the nucleic acid of claim 16.

**23.** The vector of claim 22, which has been deposited at the CNCM on July 17, 2003 under number I-3070.

**24.** A vector comprising the nucleic acid of claim 19.

**25.** A vector comprising the nucleic acid of claim 20.

**26.** A vector comprising the nucleic acid of claim 21.

**27.** The vector of claim 25 or 26, which has been deposited at the CNCM on July 17, 2003 under number I-3071.

**28.** A host cell containing the nucleic acid of claim 16 or the vector of claim 22.

**29.** A host cell containing the vector of claim 23, which has been deposited at the CNCM on July 17, 2003 under number I-3070.

**30.** A host cell containing the nucleic acid of claim 19 or the vector of claim 24.

**31.** A host cell containing the nucleic acid of claim 20 or the vector of claim 25.

**32.** A host cell containing the nucleic acid of claim 21 or the vector of claim 26.

**33.** A host cell containing the vector of claim 27, which has been deposited at the CNCM on July 17, 2003 under number I-3071.

**34.** A non-human transgenic animal that comprises the host cell of anyone of claims 28 to 33.

**35.** A transgenic plant that comprises the host cell of anyone of claims 28 to 33.

**36.** The non-human transgenic animal of claim 34 which is a mammal.

**37.** The non-human transgenic animal of claim 36 which is a mouse.

**38.** A method for the preparation of a transformed cell having a transmembrane anionic channel polypeptide, or a subunit or fragment thereof, comprising an anionic sensor expressed at the vicinity of its anionic channel on the cytoplasmic domain of said transmembrane anionic channel polypeptide, said method comprising the following step :

- transforming said cell with the nucleic acid of anyone of claims 19 to 21 or with the vector of anyone of claims 24 to 27.

**39.** A method for the detection of anions in a sample, comprising the following steps:

a) contacting said sample with a polypeptide according to anyone of the claims 1 to 10;
b) correlating the obtention of a fluorescence signal with the presence of anions in said sample.

**40.** A method for the determination of anion concentrations in a sample, comprising the following steps :

a) contacting said sample with a polypeptide according to anyone of claims 3 to 10 ;
b) measuring the obtained fluorescence signal ;
c) correlating said fluorescence signal obtained at step b) with an anion concentration, optionally by comparison with the fluorescence signal of a reference sample having a known anion concentration.

**41.** A method for the determination of anion concentrations in the compartment of a living cell, comprising the following steps :

a) transforming said living cell so as to obtain the expression of the polypeptide of anyone of claims 3 to 15 in said compartment ;
b) measuring the obtained fluorescence signal ;
c) correlating said fluorescence signal obtained at step b) with an anion concentration, optionally by comparison with the fluorescence signal of a reference living cell compartment having a known anion concentration.

**42.** A method of monitoring dynamic changes in anion concentrations in the compartment of a living cell, comprising the following steps :

a) transforming said living cell with the nucleic acid of anyone of claims 19 to 21 or with the vector of anyone of claims 24 to 27;
b) measuring the fluctuation of the obtained fluorescence signal ;
c) correlating said fluctuation of fluorescence signal obtained at step b) with dynamic changes in anion concentrations, optionally by comparison with the fluorescence signal of a reference living cell compartment having a known anion concentration.

**43.** The method of monitoring dynamic changes in anion concentrations in the compartment of a living cell of claim 42, wherein dynamic changes in anion concentrations are monitored on a pH basis, said step b) of measuring the fluctuation of the obtained fluorescence signal being performed at various pH values.

**44.** A method of selecting a compound capable of modifying anion concentrations in the compartment of a living cell, comprising the following steps:

a) determining the anion concentration using the method for the determination of anion concentrations in the compartment of a living cell of claim 41;
b) contacting said compound with said compartment;
c) determining the anion concentration using the same method as in step a);
d) selecting the compound when the anion concentration determined at step c) is different from the anion concentration determined at step a).

**45.** A method of selecting a compound capable of modifying anion concentrations in the compartment of a living cell, comprising the following steps :

a) monitoring the dynamic changes in anion concentration using the method of monitoring dynamic changes in anion concentrations in the compartment of a living cell of claim 42 or 43 ;
b) contacting said compound with said compartment;
c) monitoring the dynamic changes in anion concentrations using the same method as in step a) ;
d) selecting the compound when the dynamic changes determined at step c) are different from the dynamic changes determined at step a).

**46.** The method of anyone of claims 41 to 45, wherein said compartment is the intracellular compartment or the subcellular compartment.

**47.** The method of anyone of claims 38 to 46, wherein the anion concentrations are halide, thiocyanate (SCN-) or nitrate ($NO_3$-) ion concentrations.

**48.** The method of claim 47, wherein the halide ion is selected from the group consisting of chloride, iodure, and bromure ions.

**49.** The method of anyone of claims 38 to 48, wherein said cell is an eukaryotic cell, preferably a mammal cell or a plant cell.

**50.** A polypeptide having the sequence SEQ ID No. 4.

**51.** A nucleic acid encoding the polypeptide of claim 50.

**52.** The nucleic acid of claim 51, having the sequence SEQ ID No. 10.

**53.** Use of the polypeptide of claim 50 as a linker for the operable linkage between two fluorescent proteins involved in FRET.

**54.** A sensing fluorescent microsphere comprising a polypeptide of anyone of claims 1 to 10 associated with the surface of said microsphere.

**55.** Use of the sensing fluorescent microsphere of claim 54 for the detection of anions in a sample, or for the variation analysis of anion concentrations in a sample.

**56.** A kit for the detection and/or for the determination of the concentration of anions in a sample which comprises the sensing fluorescent microsphere of claim 54.

**57.** A kit for monitoring dynamic changes in anion concentrations in a living cell or in a subcellular compartment of a living cell, which comprises the polypeptide of anyone of claims 1 to 15, the nucleic acid of claim 16, the vector of claim 22, or the host cell of claim 28.

**58.** A kit for monitoring dynamic changes in anion concentrations in a living cell or in a subcellular compartment of a living cell, which comprises the polypeptide of anyone of claims 11 to 15, the nucleic acid of claim 19, the vector of claim 24, or the host cell of claim 30.

**59.** A kit for monitoring dynamic changes in anion concentrations in a living cell or in a subcellular compartment of a living cell, which comprises the polypeptide of claim 15, the nucleic acid of claim 20 or 21, the vector of anyone of claims 25 to 27, or the host cell of anyone of claims 31 to 33.

**60.** A kit for the diagnosis of a condition involving a transmembrane anionic channel, which comprises the polypeptide of anyone of claims 11 to 15, the nucleic acid of claim 19, the vector of claim 24, or the host cell of claim 30.

**61.** A kit for the diagnosis of a condition involving a transmembrane anionic channel, which comprises the polypeptide of claim 15, the nucleic acid of claim 20 or 21, the vector of anyone of claims 25 to 27, or the host cell of anyone of claims 31 to 33.

**62.** The kit for the diagnosis of a condition involving a transmembrane anionic channel of claim 60 or 61, wherein said condition is selected from the group consisting of cystic fibrosis, myotonia congenita, congenital chloride diarrhoea, inherited hypercalciuric nephrolithiasis, Bartter's and Gitelman's and hyperekplexia/startle disease.

**63.** A polypeptidic complex comprising a transmembrane ionic channel or a sub-unit or fragment thereof, and a polypeptidic biosensor which is capable to emit a specific signal correlated to an ion concentration.

**64.** The polypeptidic complex of claim 63, wherein the polypeptidic biosensor is a polypeptidic fluorescence biosensor and the specific signal is a fluorescence signal.

**65.** The polypeptidic complex of claim 64, wherein the polypeptidic fluorescence biosensor comprises two fluorescent proteins and is capable of exhibiting FRET.

**66.** The polypeptidic complex of claim 65, wherein one the two fluorescent proteins is EYFP.

**67.** The polypeptidic complex of claim 66, wherein the EYFP protein comprises at least one mutation, said mutation being selected in the group consisting of I152L, V163S and H148Q.

**68.** The polypeptidic complex of claim 67, wherein the EYFP protein comprises two mutations selected in said group.

**69.** The polypeptidic complex of claim 68, wherein said polypeptidic complex comprises the polypeptide of anyone of claims 1 to 15.

**70.** A method for the preparation of a transformed cell having a transmembrane ionic channel polypeptide, or a sub-unit or fragment thereof, comprising a ionic sensor expressed at the vicinity of its ionic channel on the cytoplasmic domain of said transmembrane ionic channel polypeptide, said method comprising the following step :

- transforming said cell so as to obtain the expression of the polypeptide of anyone of claims 63 to 69 in said cell.

**71.** A method for the determination of ion concentrations in the compartment of a living cell, comprising the following steps :

a) transforming said living cell so as to obtain the expression of the polypeptidic complex of anyone of claims 63 to 69 in said compartment;
b) measuring the obtained fluorescence signal ;
c) correlating said fluorescence signal obtained at step b) with an ion concentration, optionally by comparison with the fluorescence signal of a reference living cell compartment having a known ion concentration.

**72.** A method of monitoring dynamic changes in ion concentrations in the compartment of a living cell, comprising the following steps :

a) transforming said living cell so as to obtain the expression of the polypeptidic complex of anyone of claims 63 to 69 in said compartment;
b) measuring the fluctuation of the obtained fluorescence signal ;
c) correlating said fluctuation of fluorescence signal obtained at step b) with dynamic changes in ion concentrations, optionally by comparison with the fluorescence signal of a reference living cell compartment having a known ion concentration.

**73.** A method of selecting a compound capable of modifying ion concentrations in the compartment of a living cell, comprising the following steps:

a) determining the ion concentration using the method for the determination of ion concentrations in the compartment of a living cell of claim 71;
b) contacting said compound with said compartment;
c) determining the ion concentration using the same method as in step a);
d) selecting the compound when the ion concentration determined at step c) is different from the ion concentration determined at step a).

**74.** A method of selecting a compound capable of modifying ion concentrations in the compartment of a living cell, comprising the following steps :

a) monitoring the dynamic changes in ion concentrations using the method of monitoring dynamic changes in ion concentrations in the compartment of a living cell according to claim 72 ;
b) contacting said compound with said compartment;
c) monitoring the dynamic changes in ion concentrations using the same method as in step a) ;
d) selecting the compound when the dynamic changes determined at step c) are different from the dynamic changes determined at step a).

**75.** A sensing fluorescent microsphere comprising a polypeptidic complex of anyone of claims 63 to 69 associated with the surface of said microsphere.

**76.** A kit for monitoring dynamic changes in ion concentrations in a living cell or in a subcellular compartment of a living cell, which comprises the polypeptidic complex of anyone of claims 63 to 69.

**77.** A kit for the diagnosis of a condition involving a transmembrane ionic channel, which comprises the polypeptidic complex of anyone of claims 63 to 69.

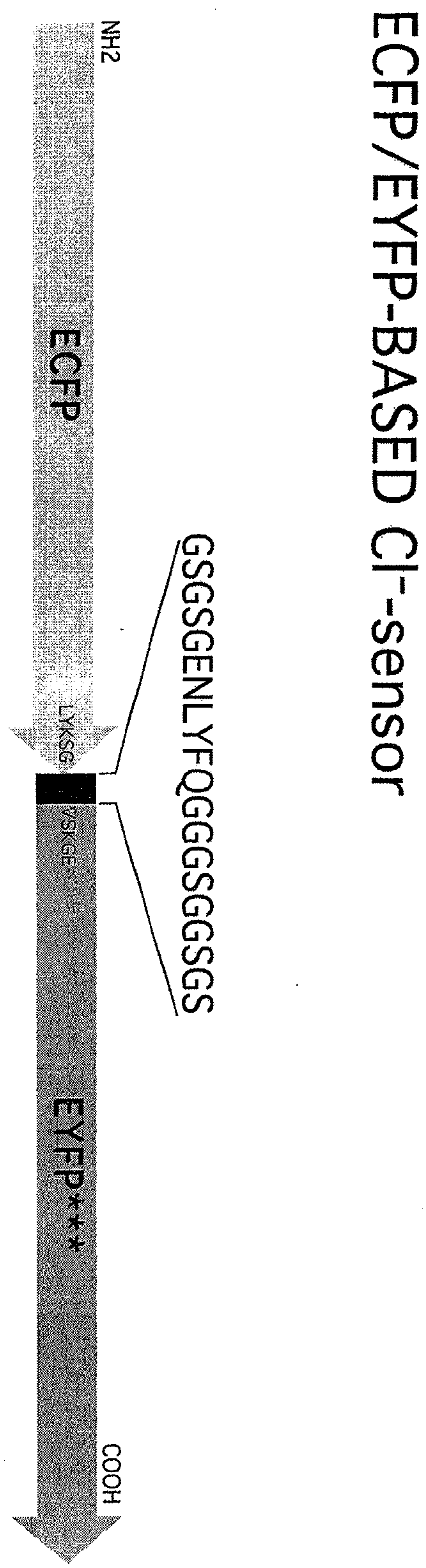
ECFP/EYFP-BASED Cl⁻-sensor
NH2
ECFP
LYKSG
GSGSGENLYFQGGGSGGSGS
Linker
VSKGE
EYFP***
COOH

FIGURE 1

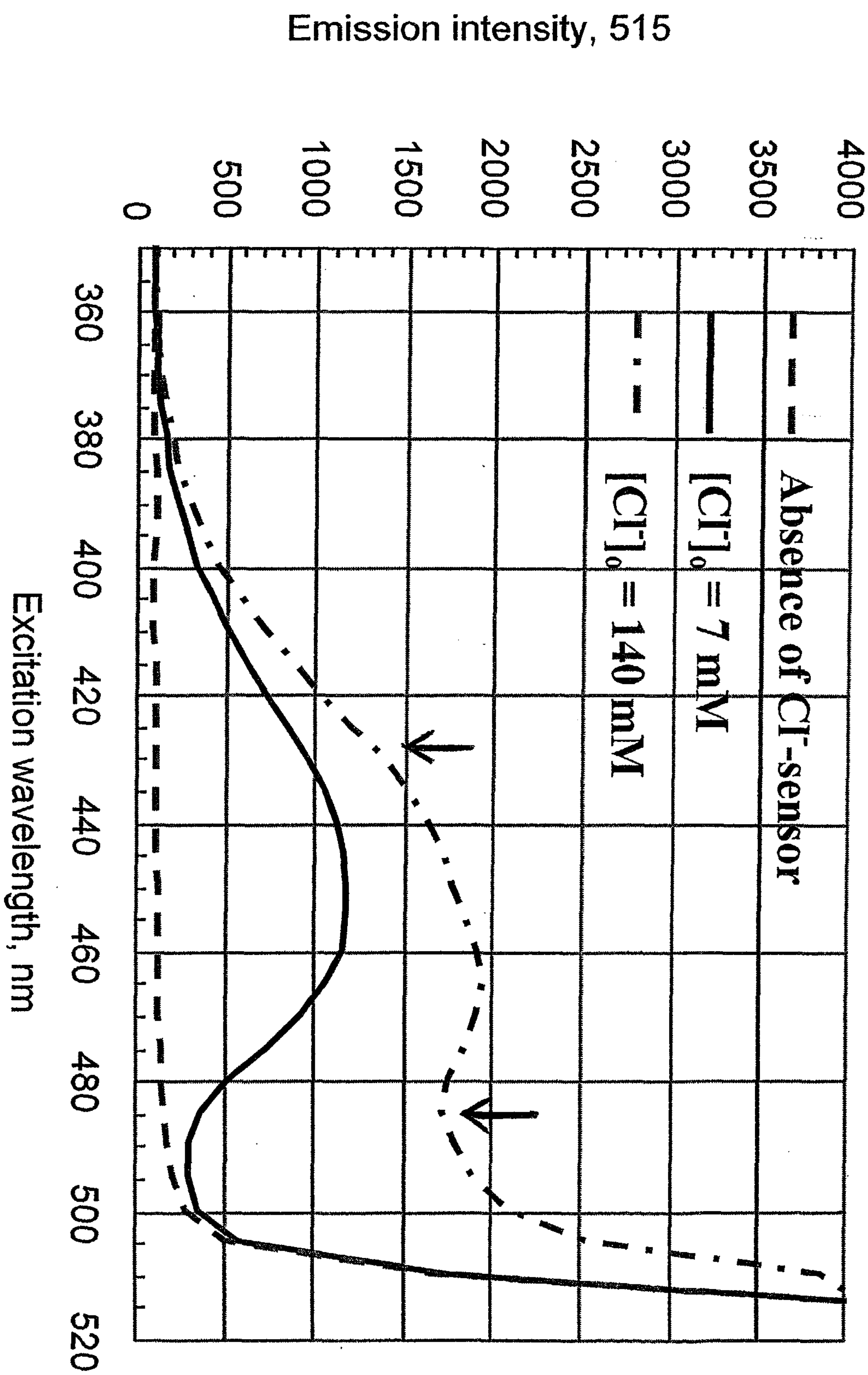
Emission intensity, 515
0
500
1000
1500
2000
2500
3000
3500
4000
360
380
400
420
440
460
480
500
520
Excitation wavelength, nm
Absence of Cl⁻-sensor
[Cl⁻]₀ = 7 mM
[Cl⁻]₀ = 140 mM

FIGURE 2

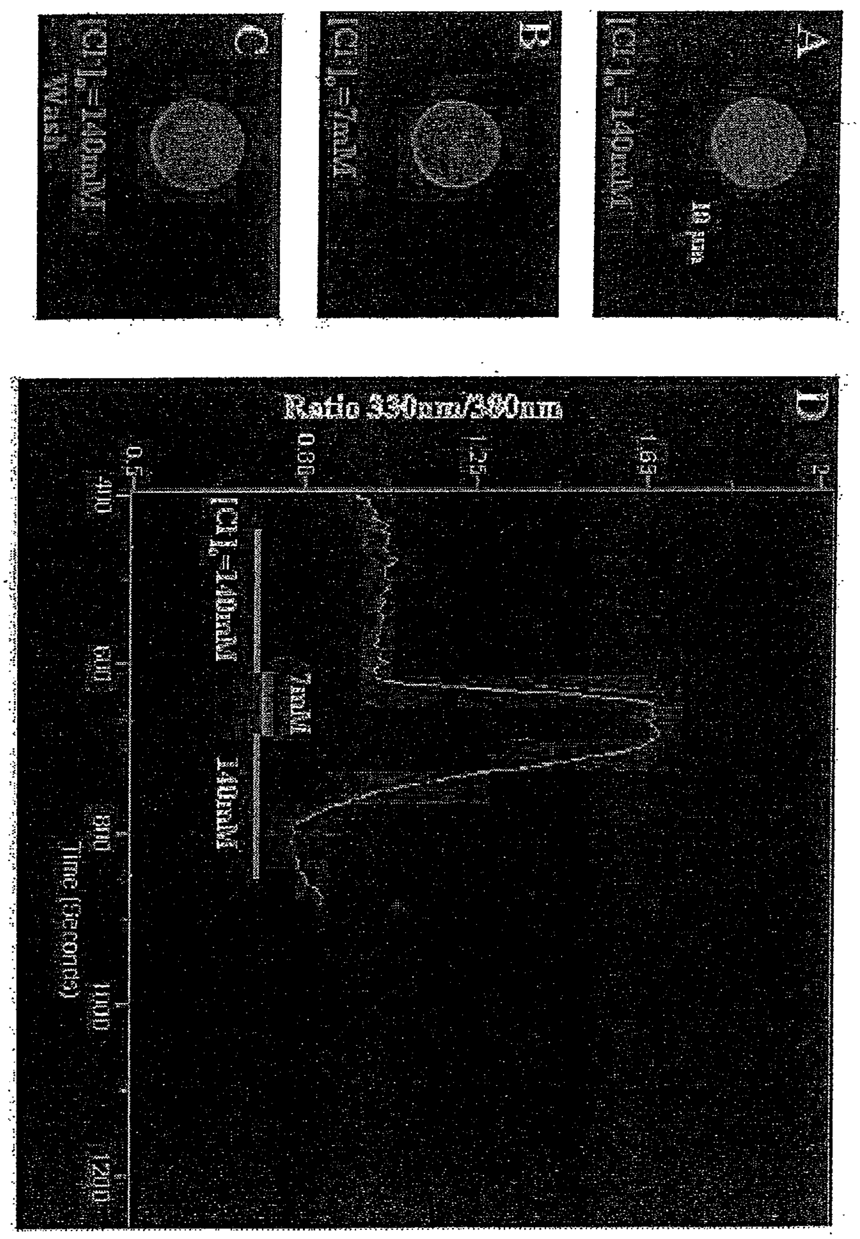
A
[Cl]o=140mM
10 μm
B
[Cl]o=7mM
C
[Cl]o=140mM
Wash
D
Ratio 330nm/380nm
0.5
0.88
1.25
1.63
2
400
600
800
1000
1200
Time (Seconds)
[Cl]o=140mM
7mM
140mM

FIGURE 3

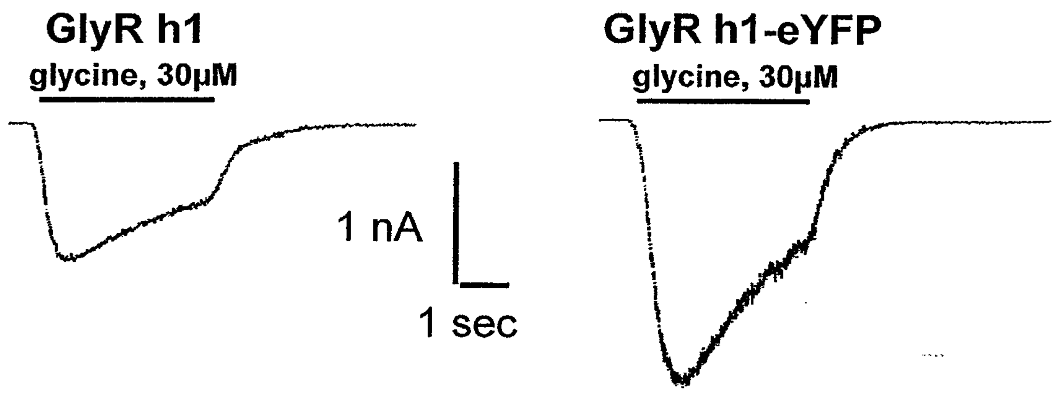


FIGURE 4A

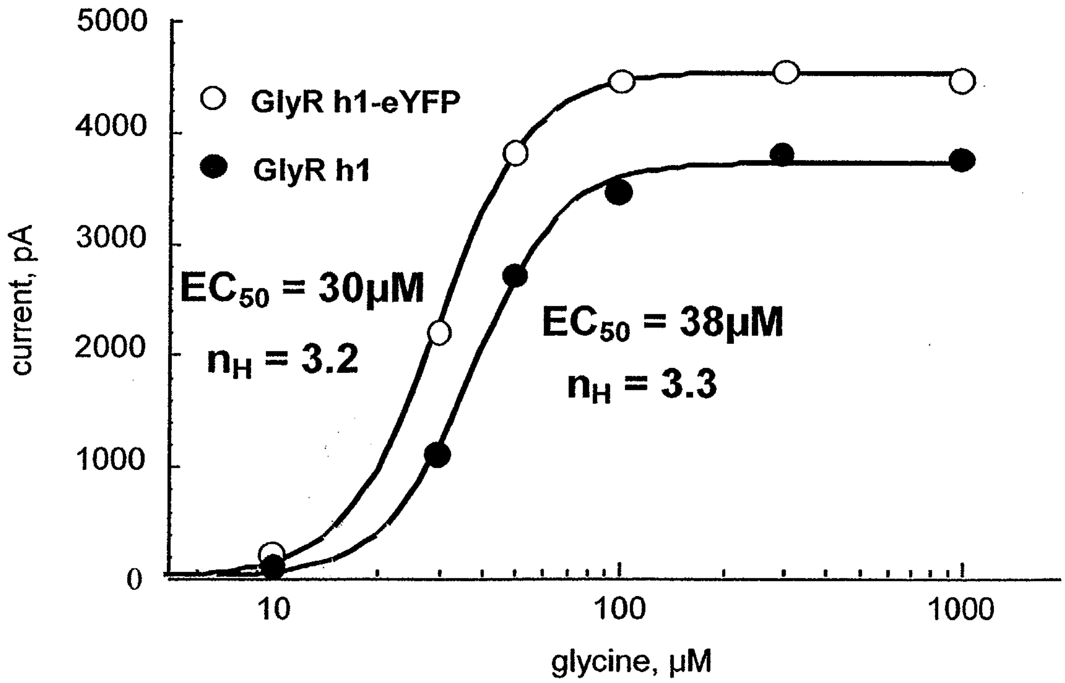


FIGURE 4B

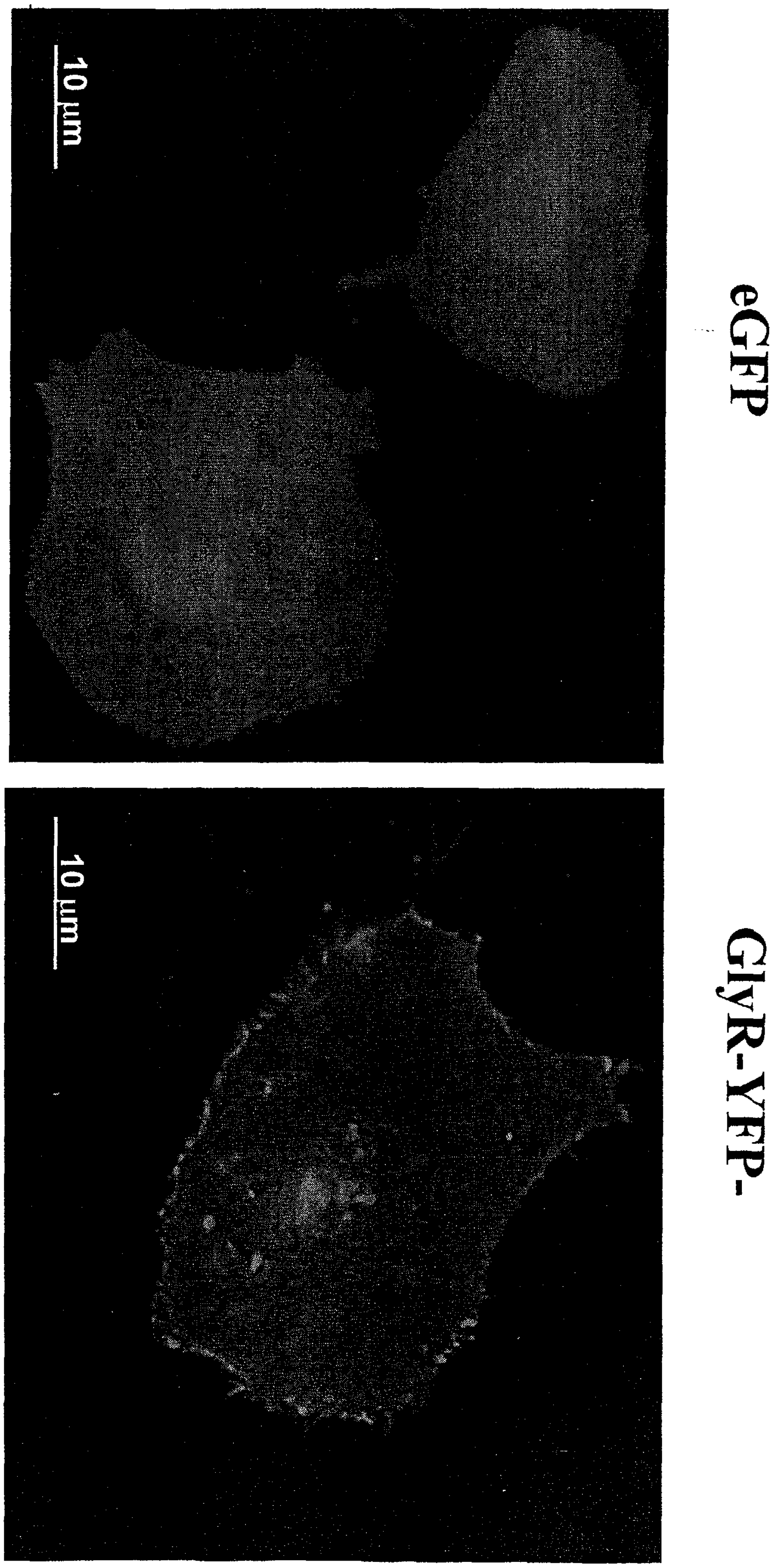


FIGURE 5

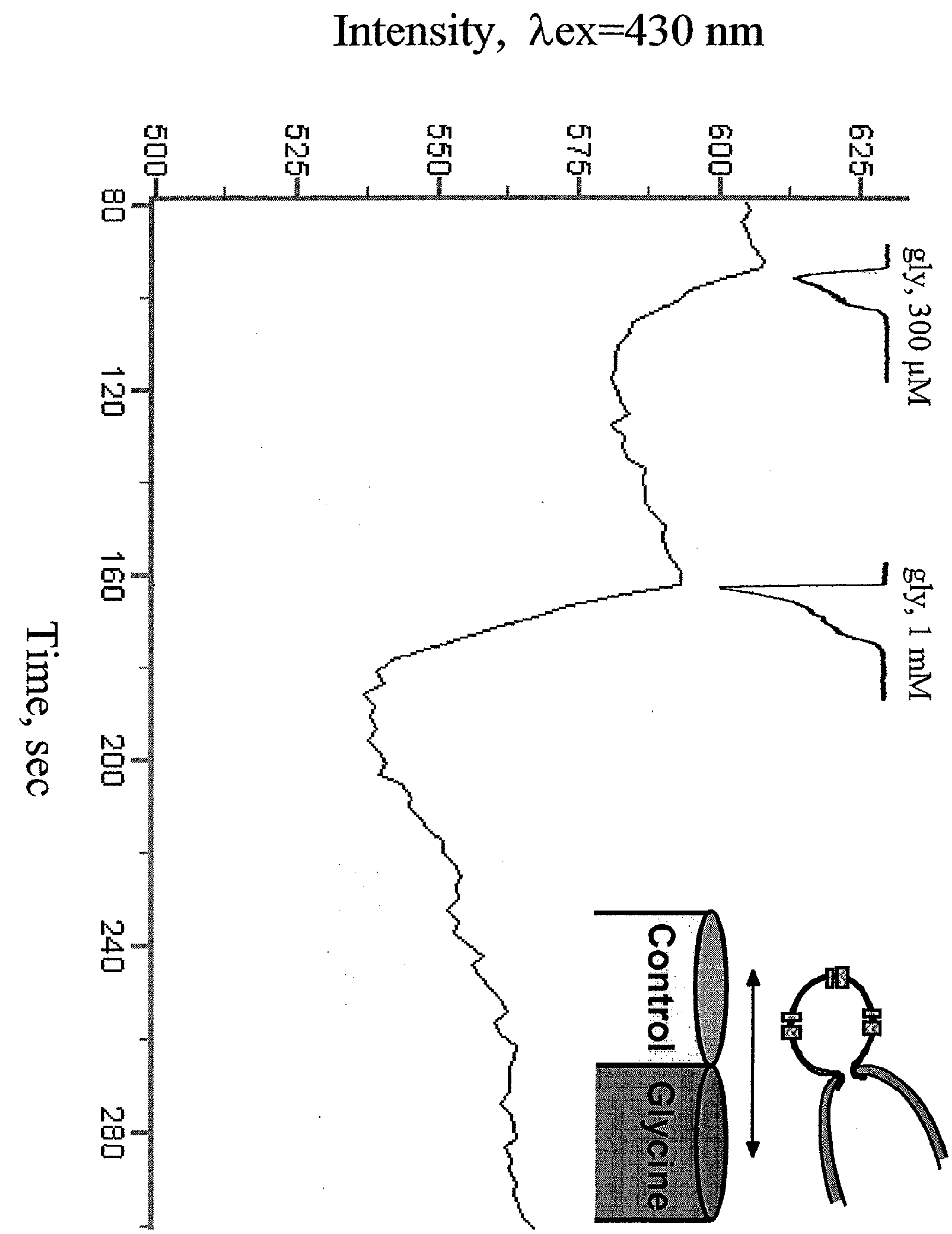
Intensity, λex=430 nm
500
525
550
575
600
625
80
120
160
200
240
280
Time, sec
gly, 300 µM
gly, 1 mM
Control
Glycine

FIGURE 6

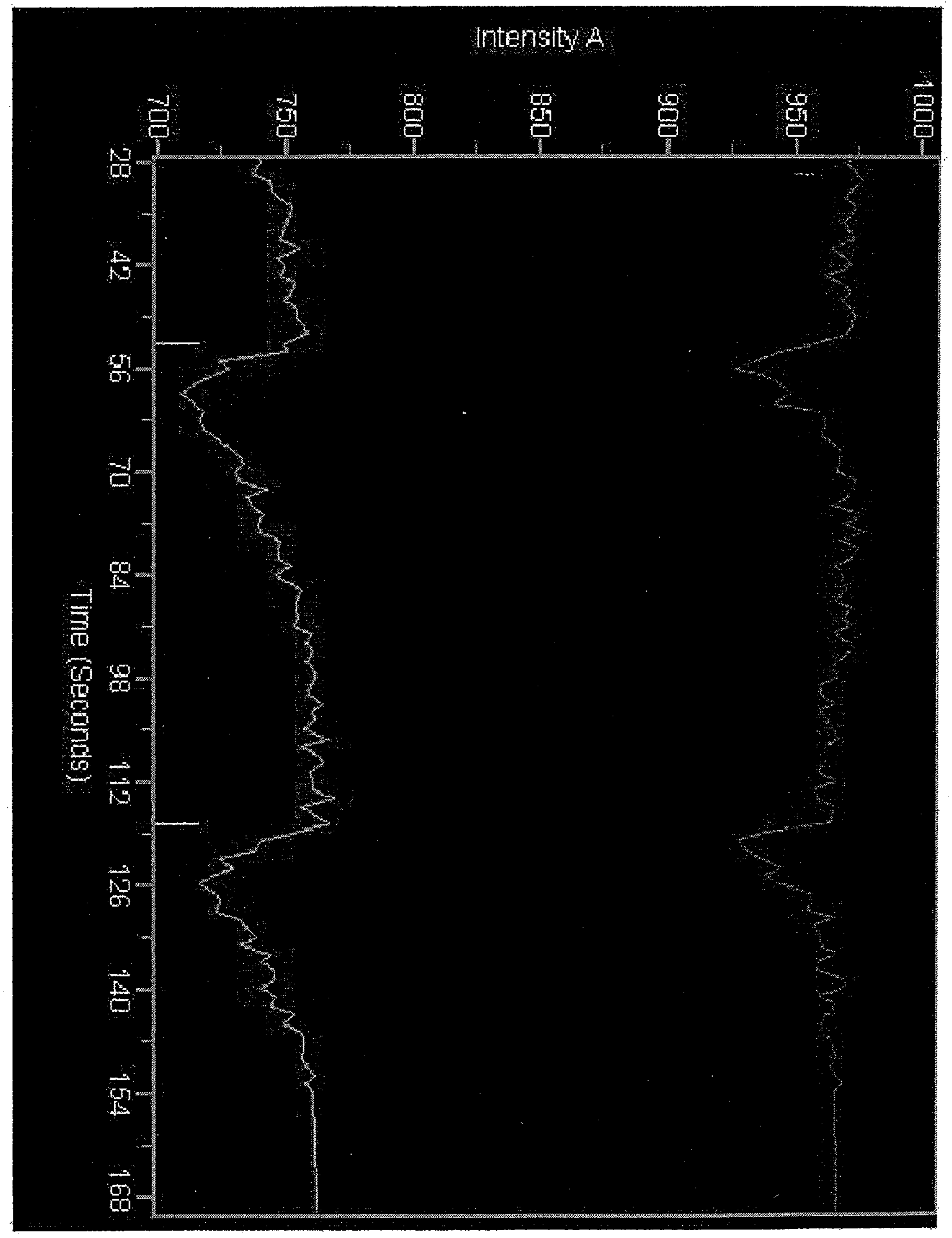
Intensity A
700
750
800
850
900
950
1000
28
42
56
70
84
98
112
126
140
154
168
Time (Seconds)

FIGURE 7

European Patent Office

# EUROPEAN SEARCH REPORT

Application Number
EP 03 29 2246

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X | GALIETTA LJV ET AL: "Green fluorescent protein-based halide indicators with improved chloride and iodide affinities" FEBS LETTERS, ELSEVIER SCIENCE PUBLISHERS, AMSTERDAM, NL, vol. 499, no. 3, 22 June 2001 (2001-06-22), pages 220-224, XP004247019 ISSN: 0014-5793 * the whole document * --- | 1-77 | C12N5/10 C12N15/82 A01H5/00 A01K67/027 C07K14/405 C07K19/00 C07K7/04 C12N15/62 G01N33/84 G01N33/542 |
| D,X | ISSHIKI MASASHI ET AL: "A molecular sensor detects signal transduction from caveolae in living cells." THE JOURNAL OF BIOLOGICAL CHEMISTRY. UNITED STATES 8 NOV 2002, vol. 277, no. 45, 8 November 2002 (2002-11-08), pages 43389-43398, XP002267649 ISSN: 0021-9258 * the whole document * --- | 63-67, 70-74 | |
| | | | TECHNICAL FIELDS SEARCHED (Int.Cl.7) |
| D,X | KUNER THOMAS ET AL: "A genetically encoded ratiometric neurotechnique indicator for chloride: Capturing chloride transients in cultured hippocampal neurons" NEURON, vol. 27, no. 3, September 2000 (2000-09), pages 447-459, XP002267650 ISSN: 0896-6273 * page 453, column 2, line 13 - line 18 * --- -/-- | 63-67, 70-74 | C07K C12N G01N |

The present search report has been drawn up for all claims

| Place of search | Date of completion of the search | Examiner |
|---|---|---|
| THE HAGUE | 3 February 2004 | Kools, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

European Patent Office

# EUROPEAN SEARCH REPORT

Application Number
EP 03 29 2246

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,A | WACHTER REBEKKA M ET AL: "Crystallographic and energetic analysis of binding of selected anions to the yellow variants of green fluorescent protein" JOURNAL OF MOLECULAR BIOLOGY, vol. 301, no. 1, 4 August 2000 (2000-08-04), pages 157-171, XP002267651 ISSN: 0022-2836 * the whole document * --- | 1-77 | |
| A | US 6 140 132 A (LLOPIS JUAN ET AL) 31 October 2000 (2000-10-31) * the whole document * --- | 1-77 | |
| A | DATABASE BIOSIS 'Online! BIOSCIENCES INFORMATION SERVICE, PHILADELPHIA, PA, US; Society for Neuroscience, Abstract No 208,9, 7 November 2002 (2002-11-07) KUNER T. ET AL.: "DEVELOPMENTAL PROFILE OF INTRACELLULAR CHLORIDE IN CORTICAL NEURONS DETERMINED IN CLOMELEON INDICATOR MOUSE LINES." Database accession no. PREV200300282956 XP002267652 * the whole document * ----- | 34 | TECHNICAL FIELDS SEARCHED (Int.Cl.7) |

The present search report has been drawn up for all claims

2

| Place of search | Date of completion of the search | Examiner |
|---|---|---|
| THE HAGUE | 3 February 2004 | Kools, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03 82 (P04C01)

## ANNEX TO THE EUROPEAN SEARCH REPORT ON EUROPEAN PATENT APPLICATION NO. EP 03 29 2246

This annex lists the patent family membersrelating to the patent documents cited in the above-mentioned European search report.
The members are as contained in the European Patent Office EDP file on
The European Patent Office is in no way liable for these particulars which are merely given for the purpose of information.

03-02-2004

| Patent document cited in search report | Publication date | Patent family member(s) | Publication date |
|---|---|---|---|
| US 6140132 A | 31-10-2000 | WO 9964592 A2 | 16-12-1999 |
| | | US 6150176 A | 21-11-2000 |
| | | US 2003212265 A1 | 13-11-2003 |
| | | US 6608189 B1 | 19-08-2003 |
| | | US 6627449 B1 | 30-09-2003 |

EPO FORM P0459

For more details about this annex : see Official Journal of the European Patent Office, No. 12/82